# EUROPEAN PATENT APPLICATION

(11) **EP 4 245 851 A1**
(43) Date of publication of application: **20.09.2023**
(21) Application number: 23159026.6
(22) Date of filing: 28.02.2023
(51) Int. Cl.: C12N 9/10, C12N 15/82

(54) **MUTANT CIS-PRENYLTRANSFERASE (CPT) FAMILY PROTEIN, METHOD FOR PRODUCING POLYISOPRENOID, VECTOR, TRANSGENIC PLANT, METHOD FOR PRODUCING PNEUMATIC TIRE, AND METHOD FOR PRODUCING RUBBER PRODUCT**

(30) Priority: 02.03.2022 JP 2022031814
(71) Applicant: Sumitomo Rubber Industries, Ltd., Kobe-shi, Hyogo 651-0072 (JP); Tohoku University, Sendai-shi, Miyagi 980-8577 (JP); Kanazawa University, Kanazawa-shi Ishikawa 920-1192 (JP); National University Corporation Saitama University, Saitama City, Saitama 338-8570 (JP)
(72) Inventor: YAMAGUCHI, Haruhiko, Kobe-shi, Hyogo, 651-0072 (JP); INOUE, Yukino, Kobe-shi, Hyogo, 651-0072 (JP); TAKAHASHI, Seiji, Sendai-shi, Miyagi, 980-8577 (JP); NAKAYAMA, Toru, Sendai-shi, Miyagi, 980-8577 (JP); YAMASHITA, Satoshi, Kanazawa-shi, Ishikawa, 9201192 (JP); TOZAWA, Yuzuru, Saitama City, Saitama, 338-8570 (JP)
(74) Representative: Winter, Brandl - Partnerschaft mbB

(57) **Abstract**

Provided are a mutant cis-prenyltransferase (CPT) family protein and a method for producing a polyisoprenoid, which enable the production of a high molecular weight polyisoprenoid. Included is a mutant cis-prenyltransferase (CPT) family protein obtained by mutating the amino acid sequence of a N-terminal region of a cis-prenyltransferase (CPT) family protein not found on rubber particles to be identical or similar to the amino acid sequence of a N-terminal region of a cis-prenyltransferase (CPT) family protein found on rubber particles.

## Description

### TECHNICAL FIELD

The present invention relates to a mutant cis-prenyltransferase (CPT) family protein, a method for producing a polyisoprenoid, a vector, a transgenic plant, a method for producing a pneumatic tire, and a method for producing a rubber product.

### BACKGROUND ART

It is known for a cis-prenyltransferase (CPT) family protein of human origin that the interaction between the N-terminus of the CPT family protein (hereinafter, also referred to simply as CPT) and a Nogo-B receptor (NgBR) family protein (corresponding to HRBP derived from rubber trees) is important for the CPT activity.

It is also known that a cis-prenyltransferase (CPT) family protein found on rubber particles (hereinafter, also referred to simply as rubber synthase) loses its enzymatic activity when it is a N-terminus-deficient rubber synthase. In other words, the N-terminus of the rubber synthase is known to be a site that is important for enzymatic activity. However, conventional techniques have room for improvement in producing a high molecular weight polyisoprenoid.

### SUMMARY OF INVENTION

### TECHNICAL PROBLEM

The present invention aims to solve the above-mentioned problem and provide a mutant cis-prenyltransferase (CPT) family protein and a method for producing a polyisoprenoid, which enable the production of a high molecular weight polyisoprenoid.

The present invention also aims to solve the above-mentioned problem and provide a vector which enables the production of a high molecular weight polyisoprenoid when it is introduced into a plant by genetic recombination techniques, as well as a transgenic plant into which the vector has been introduced.

### SOLUTION TO PROBLEM

The present inventors believe that Nogo-B receptor (NgBR) family proteins serve to assist CPT to be folded in the correct conformation. Thus, if CPT loses its interaction with NgBR, it is less likely to be folded in the correct conformation and may lose its enzymatic activity.

Studies of the present inventors have found the following.

### Finding 1: Rubber synthesis on rubber particles using wild-type CPT

It has been found that when CPT without any mutation is directly bound to rubber particles to synthesize a polyisoprenoid, no high molecular weight polyisoprenoid may be synthesized on the rubber particles, depending on the type of CPT. Particularly when a short- or medium-chain isoprenoid synthase is used which inherently does not bind to the membrane, there is, in the first place, a problem in that it cannot bind to the membrane.

### Finding 2: Polyisoprenoid synthesis on membrane particles using enzyme obtained by fusing CPT to peptide (or enzyme) that assists membrane binding

As a solution to the problem of the finding 1, the inventors tried to fuse a peptide or enzyme that assists membrane-binding ability (e.g., a membrane-binding part of a lipid droplet-associated protein or SRPP capable of binding to rubber particles) to the N-terminal or C-terminal side of CPT. However, although this method solves the membrane-binding ability problem of the enzyme, it leaves the possibility that the enzyme may not be correctly embedded in the membrane or that the fusion protein may inhibit the interaction with NgBR which is considered to be important for the enzymatic activity.

The present inventors have made extensive studies and arrived at the idea that the synthesis of a high molecular weight polyisoprenoid on rubber particles requires: correct folding of CPT (guideline a), binding thereof to the membrane (guideline b), and correct (deep) incorporation thereof into the membrane (guideline c).

As a result of extensive studies, the present inventors have found that the technique of the finding 1 faces challenges with respect to the guidelines b and c when CPT not naturally found on rubber particles is used, while the technique of the finding 2 can solve the guideline b by fusion to a membrane-binding peptide but faces challenges with respect to the guideline a (when the peptide is fused to the N-terminal side, the enzyme is not successfully folded), and the guideline c (when the peptide is fused to the C-terminal side, the enzyme is not correctly incorporated into the membrane) due to the negative effect of the peptide fusion.

While investigating the relationship between the terminal structure of CPT and the interaction with NgBR, the present inventors have discovered that a change in the N-terminal structure of CPT that is not interactive with NgBR may impart the ability to interact with NgBR to the CPT. The present inventors also have discovered that, when CPT not interactive with NgBR becomes interactive with NgBR, the molecular weight of the product's chain length in polyisoprenoid synthesis on rubber particles may be increased.

Moreover, the present inventors have found that the production of a high molecular weight polyisoprenoid may be enabled by swapping the N-terminal sequence of CPT not found on rubber particles with the N-terminal sequence of CPT found on rubber particles, rather than by fusing the N-terminal sequence of CPT found on rubber particles to the N-terminus of the former CPT. These findings have led to the completion of the present invention.

Specifically, the present invention relates to a mutant cis-prenyltransferase (CPT) family protein, obtained by mutating an amino acid sequence of a N-terminal region of a cis-prenyltransferase (CPT) family protein not found on rubber particles to be identical or similar to an amino acid sequence of a N-terminal region of a cis-prenyltransferase (CPT) family protein found on rubber particles. Hereinafter, this invention is taken as a first aspect of the present invention and may be referred to as the first invention.

### ADVANTAGEOUS EFFECTS OF INVENTION

The mutant cis-prenyltransferase (CPT) family protein according to the first invention is obtained by mutating the amino acid sequence of a N-terminal region of a cis-prenyltransferase (CPT) family protein not found on rubber particles to be identical or similar to the amino acid sequence of a N-terminal region of a cis-prenyltransferase (CPT) family protein found on rubber particles. This mutant cis-prenyltransferase (CPT) family protein enables the production of a higher molecular weight polyisoprenoid than that produced with the unmutated cis-prenyltransferase (CPT) family protein. Thus, a method for producing a polyisoprenoid using the mutant cis-prenyltransferase (CPT) family protein enables the production of a higher molecular weight polyisoprenoid than that produced with the unmutated cis-prenyltransferase (CPT) family protein.

A method for producing a pneumatic tire according to the first invention includes: producing a polyisoprenoid by the method for producing a polyisoprenoid of the first invention; kneading the polyisoprenoid with an additive to obtain a kneaded mixture; building a green tire from the kneaded mixture; and vulcanizing the green tire. With this method, which produces a pneumatic tire from a high molecular weight polyisoprenoid, it is possible to use plant resources effectively to environmentally friendly produce a pneumatic tire having excellent performance.

A method for producing a rubber product according to the first invention includes: producing a polyisoprenoid by the method for producing a polyisoprenoid of the first invention; kneading the polyisoprenoid with an additive to obtain a kneaded mixture; forming a raw rubber product from the kneaded mixture; and vulcanizing the raw rubber product. With this method, which produces a rubber product from a high molecular weight polyisoprenoid, it is possible to use plant resources effectively to environmentally friendly produce a rubber product having excellent performance.

A vector according to a second aspect of the present invention (hereinafter, also referred to as the second invention) contains a gene coding for the mutant cis-prenyltransferase (CPT) family protein of the first invention. Then, when the vector is introduced into a plant, the gene coding for the mutant cis-prenyltransferase (CPT) family protein of the first invention in the vector will be expressed to enable the production of a higher molecular weight polyisoprenoid in the plant as compared to before the gene recombination.

A method for producing a pneumatic tire according to the second invention includes: producing a polyisoprenoid using a transgenic plant produced by introducing the vector of the second invention into a plant; kneading the polyisoprenoid with an additive to obtain a kneaded mixture; building a green tire from the kneaded mixture; and vulcanizing the green tire. With this method, which produces a pneumatic tire from a high molecular weight polyisoprenoid produced by a transgenic plant that is enabled to produce a higher molecular weight polyisoprenoid as compared to before the gene recombination, it is possible to use plant resources effectively to environmentally friendly produce a pneumatic tire having excellent performance.

A method for producing a rubber product according to the second invention includes: producing a polyisoprenoid using a transgenic plant produced by introducing the vector of the second invention into a plant; kneading the polyisoprenoid with an additive to obtain a kneaded mixture; forming a raw rubber product from the kneaded mixture; and vulcanizing the raw rubber product. With this method, which produces a rubber product from a high molecular weight polyisoprenoid produced by a transgenic plant that is enabled to produce a higher molecular weight polyisoprenoid as compared to before the gene recombination, it is possible to use plant resources effectively to environmentally friendly produce a rubber product having excellent performance.

### BRIEF DESCRIPTION OF DRAWINGS

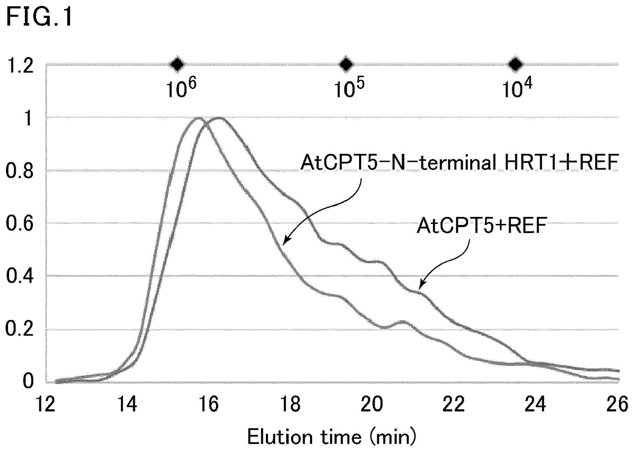

FIG. 1 is a graph that shows the measured molecular weight distributions of the polyisoprenoids synthesized in Example 1 and Comparative Example 1.

### DESCRIPTION OF EMBODIMENTS

Herein, the first invention and the second invention are also referred collectively to as the present invention. The first invention will be described first, and the second invention will be described later.

### (First Invention)

The mutant cis-prenyltransferase (CPT) family protein of the first invention is obtained by mutating the amino acid sequence of a N-terminal region of a cis-prenyltransferase (CPT) family protein not found on rubber particles to be identical or similar to the amino acid sequence of a N-terminal region of a cis-prenyltransferase (CPT) family protein found on rubber particles. This mutant cis-prenyltransferase (CPT) family protein enables the production of a higher molecular weight polyisoprenoid than that produced with the unmutated cis-prenyltransferase (CPT) family protein. Thus, a method for producing a polyisoprenoid using the mutant cis-prenyltransferase (CPT) family protein enables the production of a higher molecular weight polyisoprenoid than that produced with the unmutated cis-prenyltransferase (CPT) family protein.

Herein, the N-terminus of a protein refers to the amino terminus of the polypeptide chain, and the C-terminus of a protein refers to the carboxy terminus of the polypeptide chain.

The reason why the present invention provides the aforementioned advantageous effect is believed to be as follows. As described earlier, the present inventors have focused on the N-terminus of CPT. Although other studies have also focused on the N-terminus of CPT, the N-terminus was only regarded to be necessary just for maintaining the activity, and there is no study suggesting the relationship with the change in the product's chain length. Thus, the relationship between the N-terminus of CPT and the change in the product's chain length has not been focused on.
(1) Wild-type HRT1 (a CPT family protein found on rubber particles derived from *Hevea brasiliensis* (rubber synthase)), when bound to rubber particles, showed activity with no problem.
(2) N-terminus-deficient HRT1 was confirmed to bind to rubber particles, but no enzymatic activity was confirmed. This is consistent with the previous findings. Moreover, its interaction with NgBR also decreases.

In contrast, mutation of the N-terminus of CPT not found on rubber particles had a great impact.
(3) When wild-type AtCPT5 (CPT not found on rubber particles derived from *Arabidopsis thaliana*) was bound to rubber particles, long isoprene chains were also synthesized as well as the original product (C₃₅ polyisoprenoid).
(4) When mutated AtCPT5 with N-terminal HRT1 (AtCPT5 in which the N-terminus has been substituted with the N-terminus of HRT1) was bound to rubber particles, the product had a higher molecular weight. Moreover, it became interactive with HRBP.

A comparison between the result (3) and the result (4) revealed that the N-terminus of HRT1 also interacts with NgBR. This is also known in conventional CPTs, but the increase in the molecular weight of the product in the result (4) is a new discovery.

The effect of the N-terminus on increasing the molecular weight of the product is believed to be as follows.

Although the interaction between CPT and NgBR is considered to be involved in the folding of CPT, AtCPT5 originally forms an active folding structure without interacting with NgBR, and thus the folding effect is unlikely to cause an increase in the molecular weight.

Meanwhile, as NgBR is known to be bound to a membrane (rubber particles), when CPT interacts with NgBR, it is expected to come closer to the membrane (rubber particles) or more deeply bind to the membrane. This effect is believed to affect the increase in the molecular weight of the polyisoprenoid.

Accordingly, when a mutant CPT family protein obtained by mutating (substituting) a N-terminal region of a CPT family protein not found on rubber particles to be identical or similar to a N-terminal region of a CPT family protein found on rubber particles is used, the mutant CPT may come closer to a membrane (rubber particles) due to the interaction between the CPT and NgBR, resulting in a product having a higher molecular weight.

Thus, as the mutant CPT family protein according to the first invention is obtained by mutating the amino acid sequence of a N-terminal region of a CPT family protein not found on rubber particles to be identical or similar to the amino acid sequence of a N-terminal region of a CPT family protein found on rubber particles, such a mutant CPT family protein enables the production of a higher molecular weight polyisoprenoid than that produced with the unmutated CPT family protein. Then, a method for producing a polyisoprenoid using the mutant CPT family protein enables the production of a higher molecular weight polyisoprenoid than that produced with the unmutated CPT family protein.

### <Mutant cis-prenyltransferase (CPT) family protein>

The mutant cis-prenyltransferase (CPT) family protein of the present invention is obtained by mutating the amino acid sequence of a N-terminal region of a cis-prenyltransferase (CPT) family protein not found on rubber particles to be identical or similar to the amino acid sequence of a N-terminal region of a cis-prenyltransferase (CPT) family protein found on rubber particles.

Specifically, the mutant CPT family protein of the present invention is a mutant CPT family protein obtained by substituting the amino acid sequence of a N-terminal region of a cis-prenyltransferase (CPT) family protein not found on rubber particles with an amino acid sequence that is identical or similar to the amino acid sequence of a N-terminal region of a cis-prenyltransferase (CPT) family protein found on rubber particles.

The amino acid sequence of the N-terminal region of the cis-prenyltransferase (CPT) family protein not found on rubber particles is preferably a N-terminus-containing amino acid sequence within a section from the N-terminus of the protein to a first β strand (β strand 1) of a β sheet, more preferably an amino acid sequence consisting of the amino acid sequence of a section from the N-terminus of the protein to the β strand 1. Likewise, the amino acid sequence of the N-terminal region of the cis-prenyltransferase (CPT) family protein found on rubber particles is preferably a N-terminus-containing amino acid sequence within a section from the N-terminus of the protein to the β strand 1, more preferably an amino acid sequence consisting of the amino acid sequence of a section from the N-terminus of the protein to the β strand 1.

Herein, the term "β strand 1" refers to a β strand that is closest to the N-terminus among the β strands which constitute a β sheet structure located at the core of the structure of CPT.

Also, herein, the term "section from the N-terminus to the β strand 1" refers to a section from the N-terminus to one amino acid upstream of the amino acid sequence forming the β strand 1.

Herein, the amino acid sequence of the N-terminal region is a continuous amino acid sequence.

The amino acid sequence of the N-terminal region of the cis-prenyltransferase (CPT) family protein not found on rubber particles is preferably a N-terminus-containing amino acid sequence within 100 amino acids downstream of the N-terminus of the protein, more preferably a N-terminus-containing amino acid sequence within 90 amino acids downstream of the N-terminus of the protein, still more preferably a N-terminus-containing amino acid sequence within 85 amino acids downstream of the N-terminus of the protein, particularly preferably a N-terminus-containing amino acid sequence within 80 amino acids downstream of the N-terminus of the protein, most preferably a N-terminus-containing amino acid sequence within 75 amino acids downstream of the N-terminus of the protein.

The amino acid sequence of the N-terminal region of the cis-prenyltransferase (CPT) family protein not found on rubber particles corresponds to, for example, positions 1 to 72 of the amino acid sequence of AtCPT5 derived from *Arabidopsis thaliana* represented by SEQ ID NO:3, or positions 1 to 34 of the amino acid sequence of HbCPT5 derived from *Hevea brasiliensis* represented by SEQ ID NO:4.

Likewise, the amino acid sequence of the N-terminal region of the cis-prenyltransferase (CPT) family protein found on rubber particles is preferably a N-terminus-containing amino acid sequence within 70 amino acids downstream of the N-terminus of the protein, more preferably a N-terminus-containing amino acid sequence within 60 amino acids downstream of the N-terminus of the protein, still more preferably a N-terminus-containing amino acid sequence within 50 amino acids downstream of the N-terminus of the protein, particularly preferably a N-terminus-containing amino acid sequence within 40 amino acids downstream of the N-terminus of the protein, most preferably a N-terminus-containing amino acid sequence within 35 amino acids downstream of the N-terminus of the protein.

The amino acid sequence of the N-terminal region of the cis-prenyltransferase (CPT) family protein found on rubber particles corresponds to, for example, positions 1 to 32 of the amino acid sequence of HRT1 derived from *Hevea brasiliensis* represented by SEQ ID NO:1.

The amino acid sequence similar to the amino acid sequence of the N-terminal region of the cis-prenyltransferase (CPT) family protein found on rubber particles is preferably an amino acid sequence having at least 80% sequence identity, more preferably at least 85% sequence identity, still more preferably at least 90% sequence identity, particularly preferably at least 95% sequence identity, most preferably at least 98% sequence identity, further most preferably at least 99% sequence identity to the amino acid sequence identical to the amino acid sequence of the N-terminal region of the cis-prenyltransferase (CPT) family protein found on rubber particles.

The mutation (substitution) of the amino acid sequence may be carried out by known techniques. Examples include homologous recombination, overlap extension PCR, in-fusion cloning, and fusion of PCR products using restriction enzymes.

The amino acid sequence of a C-terminal region of the mutant CPT family protein is preferably an amino acid sequence having at least 80% sequence identity, more preferably at least 85% sequence identity, still more preferably at least 90% sequence identity, particularly preferably at least 95% sequence identity, most preferably at least 98% sequence identity, even most preferably at least 99% sequence identity, further most preferably 100% sequence identity (no change in the C-terminal structure) to the amino acid sequence of a C-terminal region of the CPT family protein not found on rubber particles.

### <<cis-prenyltransferase (CPT) family protein found on rubber particles>>

The cis-prenyltransferase (CPT) family protein found on rubber particles may be any CPT family protein found on rubber particles and may be, for example, a cis-prenyltransferase (CPT) family protein found on rubber particles of plant origin. In particular, it is preferably a cis-prenyltransferase (CPT) family protein found on rubber particles derived from a plant of the genus *Hevea* or *Taraxacum* (particularly a plant of the genus *Hevea*), more preferably a cis-prenyltransferase (CPT) family protein found on rubber particles derived from *Hevea brasiliensis* or *Taraxacum kok-saghyz* (particularly *Hevea brasiliensis*).

Non-limiting examples of the plant include: the genus *Hevea,* e.g., *Hevea brasiliensis*; the genus *Sonchus,* e.g., *Sonchus oleraceus, Sonchus asper,* and *Sonchus brachyotus*; the genus *Solidago,* e.g., *Solidago altissima, Solidago virgaurea* subsp. *asiatica, Solidago virgaurea* subsp. *leipcarpa, Solidago virgaurea* subsp. *leipcarpa* f. *paludosa, Solidago virgaurea* subsp. *gigantea,* and *Solidago gigantea* Ait. var. *leiophylla* Fernald; the genus *Helianthus,* e.g., *Helianthus annuus, Helianthus argophyllus, Helianthus atrorubens, Helianthus debilis, Helianthus decapetalus,* and *Helianthus giganteus*; the genus *Taraxacum,* e.g., dandelion (*Taraxacum*), *Taraxacum venustum* H. Koidz, *Taraxacum hondoense* Nakai, *Taraxacum platycarpum* Dahls, *Taraxacum japonicum, Taraxacum officinale* Weber, and *Taraxacum kok-saghyz;* the genus *Ficus,* e.g., *Ficus carica, Ficus elastica, Ficus pumila* L., *Ficus erecta* Thumb., *Ficus ampelas* Burm. f., *Ficus benguetensis* Merr., *Ficus irisana* Elm., *Ficus microcarpa* L. f., *Ficus septica* Burm. f., and *Ficus benghalensis;* the genus *Parthenium,* e.g., *Parthenium argentatum, Parthenium hysterophorus,* and annual ragweed (*Ambrosia artemisiifolia*); and lettuce *(Lactuca sativa*).

Specific examples of the CPT family protein found on rubber particles include HRT1 and HRT2 which are CPT family proteins found on rubber particles derived from *Hevea brasiliensis,* CPT1 which is a CPT family protein found on rubber particles derived from *Taraxacum kok-saghyz,* and CPT3 which is a CPT family protein found on rubber particles derived from *Parthenium argentatum.* HRT1 is preferred among these.

A specific example of the CPT family protein found on rubber particles is the following protein [1]: [1] a protein having the amino acid sequence represented by SEQ ID NO:1.

It is also known that proteins having one or more amino acid substitutions, deletions, insertions, or additions relative to the original amino acid sequence can still have the inherent function. Thus, another specific example of the CPT family protein is the following protein [2] :
a protein having an amino acid sequence containing one or more amino acid substitutions, deletions, insertions, and/or additions relative to the amino acid sequence represented by SEQ ID NO:1, and having an enzyme activity that catalyzes a reaction of cis-chain elongation of an isoprenoid compound.

Here, in order to maintain the function of the CPT family protein, it preferably has an amino acid sequence containing one or more, more preferably 1 to 58, still more preferably 1 to 44, further preferably 1 to 29, particularly preferably 1 to 15, most preferably 1 to 6, even most preferably 1 to 3 amino acid substitutions, deletions, insertions, and/or additions relative to the amino acid sequence of SEQ ID NO:1.

Conservative substitutions are preferred as examples of amino acid substitutions. Specific examples include substitutions within each of the following groups in the parentheses: (glycine, alanine), (valine, isoleucine, leucine), (aspartic acid, glutamic acid), (asparagine, glutamine), (serine, threonine), (lysine, arginine), and (phenylalanine, tyrosine).

It is also known that proteins with amino acid sequences having high sequence identity to the original amino acid sequence can also have similar functions. Thus, another specific example of the CPT family protein is the following protein [3]:
a protein having an amino acid sequence with at least 80% sequence identity to the amino acid sequence represented by SEQ ID NO:1, and having an enzyme activity that catalyzes a reaction of cis-chain elongation of an isoprenoid compound.

Here, in order to maintain the function of the CPT family protein, the sequence identity to the amino acid sequence of SEQ ID NO:1 is preferably at least 85%, more preferably at least 90%, still more preferably at least 95%, particularly preferably at least 98%, most preferably at least 99%.

The sequence identity between amino acid sequences or nucleotide sequences may be determined using the algorithm BLAST [Pro. Natl. Acad. Sci. USA, 90, 5873 (1993), which is herein incorporated by reference] developed by Karlin and Altschul or FASTA [Methods Enzymol., 183, 63 (1990), which is herein incorporated by reference].

Whether it is a protein having the above enzyme activity may be determined by, for example, conventional techniques, such as by expressing a target protein in a transformant produced by introducing a gene coding for the target protein into *Escherichia coli* or other host organisms, and determining the presence or absence of the function of the target protein by the corresponding activity measuring method.

Specific examples of the gene coding for the CPT family protein found on rubber particles include the following DNAs [1] and [2]:
[1] a DNA having the nucleotide sequence represented by SEQ ID NO:2; and
[2] a DNA which hybridizes under stringent conditions to a DNA having a nucleotide sequence complementary to the nucleotide sequence represented by SEQ ID NO:2, and which codes for a protein having an enzyme activity that catalyzes a reaction of cis-chain elongation of an isoprenoid compound.

Herein, the term "hybridize" means a process in which a DNA hybridizes to a DNA having a specific nucleotide sequence or a part of the DNA. Thus, the DNA having a specific nucleotide sequence or part of the DNA may have a nucleotide sequence long enough to be usable as a probe in Northern or Southern blot analysis or as an oligonucleotide primer in polymerase chain reaction (PCR) analysis. The DNA used as a probe may have a length of at least 100 bases, preferably at least 200 bases, more preferably at least 500 bases, but it may be a DNA of at least 10 bases, preferably at least 15 bases.

Techniques to perform DNA hybridization experiments are well known. The hybridization conditions under which experiments are carried out may be determined in accordance with, for example, Molecular Cloning, 2nd ed. and 3rd ed. (2001), Methods for General and Molecular Bacteriology, ASM Press (1994), Immunology methods manual, Academic press (Molecular), each of which is herein incorporated by reference, and many other standard textbooks.

The stringent conditions may include, for example, an overnight incubation at 42°C of a DNA-immobilized filter and a DNA probe in a solution containing 50% formamide, 5× SSC (750 mM sodium chloride, 75 mM sodium citrate), 50 mM sodium phosphate (pH 7.6), 5× Denhardt's solution, 10% dextran sulfate, and 20 µg/L denatured salmon sperm DNA, followed by washing the filter for example in a 0.2× SSC solution at approximately 65°C. Less stringent conditions may also be employed. Changes in stringency may be accomplished through the manipulation of formamide concentration (lower percentages of formamide result in lower stringency), salt concentrations, or temperature.
For example, low stringent conditions include an overnight incubation at 37°C in a solution containing 6× SSCE (20× SSCE: 3 mol/L sodium chloride, 0.2 mol/L sodium dihydrogen phosphate, 0.02 mol/L EDTA, pH 7.4), 0.5% SDS, 30% formamide, and 100 µg/L denatured salmon sperm DNA, followed by washing in a 1× SSC solution containing 0.1% SDS at 50°C. In addition, to achieve even lower stringency, washes performed following hybridization may be done at higher salt concentrations (e.g., 5× SSC) in the above-mentioned low stringent conditions.

Variations in the above conditions may be accomplished through the addition or substitution of blocking reagents used to suppress background in hybridization experiments. The addition of blocking reagents may require modification of the hybridization conditions for compatibility.

The DNA capable of hybridizing under stringent conditions as described above may have a nucleotide sequence with at least 80%, preferably at least 90%, more preferably at least 95%, still more preferably at least 98%, particularly preferably at least 99% sequence identity to the nucleotide sequence of SEQ ID NO:2 as calculated using a program such as BLAST or FASTA with the parameters mentioned above.

Whether the DNA which hybridizes to the above-mentioned DNA under stringent conditions codes for a protein having a predetermined enzyme activity may be determined by conventional techniques, such as by expressing a target protein in a transformant produced by introducing a gene coding for the target protein into *Escherichia coli* or other host organisms, and determining the presence or absence of the function of the target protein by the corresponding activity measuring method.

Moreover, conventional techniques may be employed to identify the amino acid sequence and nucleotide sequence of the protein. For example, total RNA is extracted from a growing plant, the mRNA is optionally purified, and a cDNA is synthesized by a reverse transcription reaction. Subsequently, degenerate primers are designed based on the amino acid sequence of a known protein corresponding to the target protein, a DNA fragment is partially amplified by RT-PCR, and the sequence is partially identified. Then, the full-length nucleotide sequence and amino acid sequence are identified by the RACE method, for example. The RACE method (rapid amplification of cDNA ends method) refers to a method in which, when the nucleotide sequence of a cDNA is partially known, PCR is performed based on the nucleotide sequence data of such a known region to clone the unknown region extending to the cDNA terminal. This method can clone full-length cDNA by PCR without preparing a cDNA library.

Here, the degenerate primers may each preferably be prepared from a plant-derived sequence having a highly similar sequence part to the target protein.

Moreover, if the nucleotide sequence coding for the protein is known, the full-length nucleotide sequence or amino acid sequence can be identified by designing a primer containing a start codon and a primer containing a stop codon using the known nucleotide sequence, followed by performing RT-PCR using a synthesized cDNA as a template.

### <<cis-prenyltransferase (CPT) family protein not found on rubber particles>>

The cis-prenyltransferase (CPT) family protein not found on rubber particles may be any CPT family protein not found on rubber particles and may be, for example, a cis-prenyltransferase (CPT) family protein not found on rubber particles of plant origin. Here, examples of the plant include those mentioned above. In particular, the cis-prenyltransferase (CPT) family protein not found on rubber particles is preferably a cis-prenyltransferase (CPT) family protein not found on rubber particles derived from a plant of the genus *Hevea* or *Taraxacum* (particularly a plant of the genus *Hevea*), more preferably a cis-prenyltransferase (CPT) family protein not found on rubber particles derived from *Hevea brasiliensis* or *Taraxacum kok-saghyz* (particularly *Hevea brasiliensis*). It is also preferably a cis-prenyltransferase (CPT) family protein not found on rubber particles derived from *Arabidopsis thaliana.*

The CPT family protein not found on rubber particles is also preferably a protein that does not interact with a Nogo-B receptor (NgBR) family protein.

Specific examples of the CPT family protein not found on rubber particles include AtCPT4 and AtCPT5 which are CPTs not found on rubber particles derived from *Arabidopsis thaliana,* HbCPT4 and HbCPT5 which are CPTs not found on rubber particles derived from *Hevea brasiliensis,* and NDPS1 which is a CPT not found on rubber particles derived from *Solanum lycopersicum.* AtCPT5 and HbCPT5 are preferred among these, with AtCPT5 being more preferred.

Specific examples of the CPT family protein not found on rubber particles include the following protein [4]: [4] a protein having the amino acid sequence represented by SEQ ID NO:3.

It is also known that proteins having one or more amino acid substitutions, deletions, insertions, or additions relative to the original amino acid sequence can still have the inherent function. Thus, another specific example of the CPT family protein is the following protein [5] :
a protein having an amino acid sequence containing one or more amino acid substitutions, deletions, insertions, and/or additions relative to the amino acid sequence represented by SEQ ID NO:3, and having an enzyme activity that catalyzes a reaction of cis-chain elongation of an isoprenoid compound.

Here, in order to maintain the function of the CPT family protein, it preferably has an amino acid sequence containing one or more, more preferably 1 to 60, still more preferably 1 to 45, further preferably 1 to 30, particularly preferably 1 to 15, most preferably 1 to 6, even most preferably 1 to 3 amino acid substitutions, deletions, insertions, and/or additions relative to the amino acid sequence of SEQ ID NO:3.

Conservative substitutions are preferred as examples of amino acid substitutions. Specific examples include substitutions within each of the following groups in the parentheses: (glycine, alanine), (valine, isoleucine, leucine), (aspartic acid, glutamic acid), (asparagine, glutamine), (serine, threonine), (lysine, arginine), and (phenylalanine, tyrosine).

It is also known that proteins with amino acid sequences having high sequence identity to the original amino acid sequence can also have similar functions. Thus, another specific example of the CPT family protein is the following protein [6]:
a protein having an amino acid sequence with at least 80% sequence identity to the amino acid sequence represented by SEQ ID NO:3, and having an enzyme activity that catalyzes a reaction of cis-chain elongation of an isoprenoid compound.

Here, in order to maintain the function of the CPT family protein, the sequence identity to the amino acid sequence of SEQ ID NO:3 is preferably at least 85%, more preferably at least 90%, still more preferably at least 95%, particularly preferably at least 98%, most preferably at least 99%.

The sequence identity between amino acid sequences or nucleotide sequences may be determined using the algorithm BLAST [Pro. Natl. Acad. Sci. USA, 90, 5873 (1993), which is herein incorporated by reference] developed by Karlin and Altschul or FASTA [Methods Enzymol., 183, 63 (1990), which is herein incorporated by reference].

Whether it is a protein having the above enzyme activity may be determined by, for example, conventional techniques, such as by expressing a target protein in a transformant produced by introducing a gene coding for the target protein into *Escherichia coli* or other host organisms, and determining the presence or absence of the function of the target protein by the corresponding activity measuring method.

Other specific examples of the CPT family protein not found on rubber particles include the following protein [7] :
a protein having the amino acid sequence represented by SEQ ID NO:4.

It is also known that proteins having one or more amino acid substitutions, deletions, insertions, or additions relative to the original amino acid sequence can still have the inherent function. Thus, another specific examples of the CPT family protein is the following protein [8] :
a protein having an amino acid sequence containing one or more amino acid substitutions, deletions, insertions, and/or additions relative to the amino acid sequence represented by SEQ ID NO:4, and having an enzyme activity that catalyzes a reaction of cis-chain elongation of an isoprenoid compound.

Here, in order to maintain the function of the CPT family protein, it preferably has an amino acid sequence containing one or more, more preferably 1 to 73, still more preferably 1 to 55, further preferably 1 to 37, particularly preferably 1 to 18, most preferably 1 to 7, even most preferably 1 to 4 amino acid substitutions, deletions, insertions, and/or additions relative to the amino acid sequence of SEQ ID NO:4.

Conservative substitutions are preferred as examples of amino acid substitutions. Specific examples include substitutions within each of the following groups in the parentheses: (glycine, alanine), (valine, isoleucine, leucine), (aspartic acid, glutamic acid), (asparagine, glutamine), (serine, threonine), (lysine, arginine), and (phenylalanine, tyrosine).

It is also known that proteins with amino acid sequences having high sequence identity to the original amino acid sequence can also have similar functions. Thus, another specific example of the CPT family protein is the following protein [9]:
a protein having an amino acid sequence with at least 80% sequence identity to the amino acid sequence represented by SEQ ID NO:4, and having an enzyme activity that catalyzes a reaction of cis-chain elongation of an isoprenoid compound.

Here, in order to maintain the function of the CPT family protein, the sequence identity to the amino acid sequence of SEQ ID NO:4 is preferably at least 85%, more preferably at least 90%, still more preferably at least 95%, particularly preferably at least 98%, most preferably at least 99%.

The sequence identity between amino acid sequences or nucleotide sequences may be determined using the algorithm BLAST [Pro. Natl. Acad. Sci. USA, 90, 5873 (1993), which is herein incorporated by reference] developed by Karlin and Altschul or FASTA [Methods Enzymol., 183, 63 (1990), which is herein incorporated by reference].

Whether it is a protein having the above enzyme activity may be determined by, for example, conventional techniques, such as by expressing a target protein in a transformant produced by introducing a gene coding for the target protein into *Escherichia coli* or other host organisms, and determining the presence or absence of the function of the target protein by the corresponding activity measuring method.

Specific examples of the gene coding for the CPT family protein not found on rubber particles include the following DNAs [3] and [4]:
[3] a DNA having the nucleotide sequence represented by SEQ ID NO:5; and
[4] a DNA which hybridizes under stringent conditions to a DNA having a nucleotide sequence complementary to the nucleotide sequence represented by SEQ ID NO:5, and which codes for a protein having an enzyme activity that catalyzes a reaction of cis-chain elongation of an isoprenoid compound.

Herein, the term "hybridize" means a process in which a DNA hybridizes to a DNA having a specific nucleotide sequence or a part of the DNA. Thus, the DNA having a specific nucleotide sequence or part of the DNA may have a nucleotide sequence long enough to be usable as a probe in Northern or Southern blot analysis or as an oligonucleotide primer in polymerase chain reaction (PCR) analysis. The DNA used as a probe may have a length of at least 100 bases, preferably at least 200 bases, more preferably at least 500 bases, but it may be a DNA of at least 10 bases, preferably at least 15 bases.

Techniques to perform DNA hybridization experiments are well known. The hybridization conditions under which experiments are carried out may be determined in accordance with, for example, Molecular Cloning, 2nd ed. and 3rd ed. (2001), Methods for General and Molecular Bacteriology, ASM Press (1994), Immunology methods manual, Academic press (Molecular), each of which is herein incorporated by reference, and many other standard textbooks.

The stringent conditions may include, for example, an overnight incubation at 42°C of a DNA-immobilized filter and a DNA probe in a solution containing 50% formamide, 5× SSC (750 mM sodium chloride, 75 mM sodium citrate), 50 mM sodium phosphate (pH 7.6), 5× Denhardt's solution, 10% dextran sulfate, and 20 µg/L denatured salmon sperm DNA, followed by washing the filter for example in a 0.2× SSC solution at approximately 65°C. Less stringent conditions may also be employed. Changes in stringency may be accomplished through the manipulation of formamide concentration (lower percentages of formamide result in lower stringency), salt concentrations, or temperature.
For example, low stringent conditions include an overnight incubation at 37°C in a solution containing 6× SSCE (20× SSCE: 3 mol/L sodium chloride, 0.2 mol/L sodium dihydrogen phosphate, 0.02 mol/L EDTA, pH 7.4), 0.5% SDS, 30% formamide, and 100 µg/L denatured salmon sperm DNA, followed by washing in a 1× SSC solution containing 0.1% SDS at 50°C. In addition, to achieve even lower stringency, washes performed following hybridization may be done at higher salt concentrations (e.g., 5× SSC) in the above-mentioned low stringent conditions.

Variations in the above conditions may be accomplished through the addition or substitution of blocking reagents used to suppress background in hybridization experiments. The addition of blocking reagents may require modification of the hybridization conditions for compatibility.

The DNA capable of hybridizing under stringent conditions as described above may have a nucleotide sequence with at least 80%, preferably at least 90%, more preferably at least 95%, still more preferably at least 98%, particularly preferably at least 99% sequence identity to the nucleotide sequence of SEQ ID NO:5 as calculated using a program such as BLAST or FASTA with the parameters mentioned above.

Whether the DNA which hybridizes to the above-mentioned DNA under stringent conditions codes for a protein having a predetermined enzyme activity may be determined by conventional techniques, such as by expressing a target protein in a transformant produced by introducing a gene coding for the target protein into *Escherichia coli* or other host organisms, and determining the presence or absence of the function of the target protein by the corresponding activity measuring method.

Moreover, conventional techniques may be employed to identify the amino acid sequence and nucleotide sequence of the protein. For example, total RNA is extracted from a growing plant, the mRNA is optionally purified, and a cDNA is synthesized by a reverse transcription reaction. Subsequently, degenerate primers are designed based on the amino acid sequence of a known protein corresponding to the target protein, a DNA fragment is partially amplified by RT-PCR, and the sequence is partially identified. Then, the full-length nucleotide sequence and amino acid sequence are identified by the RACE method, for example. The RACE method (rapid amplification of cDNA ends method) refers to a method in which, when the nucleotide sequence of a cDNA is partially known, PCR is performed based on the nucleotide sequence data of such a known region to clone the unknown region extending to the cDNA terminal. This method can clone full-length cDNA by PCR without preparing a cDNA library.

Here, the degenerate primers may each preferably be prepared from a plant-derived sequence having a highly similar sequence part to the target protein.

Moreover, if the nucleotide sequence coding for the protein is known, the full-length nucleotide sequence and amino acid sequence can be identified by designing a primer containing a start codon and a primer containing a stop codon using the known nucleotide sequence, followed by performing RT-PCR using a synthesized cDNA as a template.

Other specific examples of the gene coding for the CPT family protein not found on rubber particles include the following DNAs [5] and [6]:
[5] a DNA having the nucleotide sequence represented by SEQ ID NO:6; and
[6] a DNA which hybridizes under stringent conditions to a DNA having a nucleotide sequence complementary to the nucleotide sequence represented by SEQ ID NO:6, and which codes for a protein having an enzyme activity that catalyzes a reaction of cis-chain elongation of an isoprenoid compound.

Herein, the term "hybridize" means a process in which a DNA hybridizes to a DNA having a specific nucleotide sequence or a part of the DNA. Thus, the DNA having a specific nucleotide sequence or part of the DNA may have a nucleotide sequence long enough to be usable as a probe in Northern or Southern blot analysis or as an oligonucleotide primer in polymerase chain reaction (PCR) analysis. The DNA used as a probe may have a length of at least 100 bases, preferably at least 200 bases, more preferably at least 500 bases, but it may be a DNA of at least 10 bases, preferably at least 15 bases.

Techniques to perform DNA hybridization experiments are well known. The hybridization conditions under which experiments are carried out may be determined in accordance with, for example, Molecular Cloning, 2nd ed. and 3rd ed. (2001), Methods for General and Molecular Bacteriology, ASM Press (1994), Immunology methods manual, Academic press (Molecular), each of which is herein incorporated by reference, and many other standard textbooks.

The stringent conditions may include, for example, an overnight incubation at 42°C of a DNA-immobilized filter and a DNA probe in a solution containing 50% formamide, 5× SSC (750 mM sodium chloride, 75 mM sodium citrate), 50 mM sodium phosphate (pH 7.6), 5× Denhardt's solution, 10% dextran sulfate, and 20 µg/L denatured salmon sperm DNA, followed by washing the filter for example in a 0.2× SSC solution at approximately 65°C. Less stringent conditions may also be employed. Changes in stringency may be accomplished through the manipulation of formamide concentration (lower percentages of formamide result in lower stringency), salt concentrations, or temperature.
For example, low stringent conditions include an overnight incubation at 37°C in a solution containing 6× SSCE (20× SSCE: 3 mol/L sodium chloride, 0.2 mol/L sodium dihydrogen phosphate, 0.02 mol/L EDTA, pH 7.4), 0.5% SDS, 30% formamide, and 100 µg/L denatured salmon sperm DNA, followed by washing in a 1× SSC solution containing 0.1% SDS at 50°C. In addition, to achieve even lower stringency, washes performed following hybridization may be done at higher salt concentrations (e.g., 5× SSC) in the above-mentioned low stringent conditions.

Variations in the above conditions may be accomplished through the addition or substitution of blocking reagents used to suppress background in hybridization experiments. The addition of blocking reagents may require modification of the hybridization conditions for compatibility.

The DNA capable of hybridizing under stringent conditions as described above may have a nucleotide sequence with at least 80%, preferably at least 90%, more preferably at least 95%, still more preferably at least 98%, particularly preferably at least 99% sequence identity to the nucleotide sequence of SEQ ID NO:6 as calculated using a program such as BLAST or FASTA with the parameters mentioned above.

Whether the DNA which hybridizes to the above-mentioned DNA under stringent conditions codes for a protein having a predetermined enzyme activity may be determined by conventional techniques, such as by expressing a target protein in a transformant produced by introducing a gene coding for the target protein into *Escherichia coli* or other host organisms, and determining the presence or absence of the function of the target protein by the corresponding activity measuring method.

Moreover, conventional techniques may be employed to identify the amino acid sequence and nucleotide sequence of the protein. For example, total RNA is extracted from a growing plant, the mRNA is optionally purified, and a cDNA is synthesized by a reverse transcription reaction. Subsequently, degenerate primers are designed based on the amino acid sequence of a known protein corresponding to the target protein, a DNA fragment is partially amplified by RT-PCR, and the sequence is partially identified. Then, the full-length nucleotide sequence and amino acid sequence are identified by the RACE method, for example. The RACE method (rapid amplification of cDNA ends method) refers to a method in which, when the nucleotide sequence of a cDNA is partially known, PCR is performed based on the nucleotide sequence data of such a known region to clone the unknown region extending to the cDNA terminal. This method can clone full-length cDNA by PCR without preparing a cDNA library.

Here, the degenerate primers may each preferably be prepared from a plant-derived sequence having a highly similar sequence part to the target protein.

Moreover, if the nucleotide sequence coding for the protein is known, the full-length nucleotide sequence and amino acid sequence can be identified by designing a primer containing a start codon and a primer containing a stop codon using the known nucleotide sequence, followed by performing RT-PCR using a synthesized cDNA as a template.

### <<Specific examples of mutant cis-prenyltransferase (CPT) family protein>>

Specific examples of the mutant CPT family protein include the following protein [A-1]:
[A-1] a protein having the amino acid sequence represented by SEQ ID NO:7.

It is also known that proteins having one or more amino acid substitutions, deletions, insertions, or additions relative to the original amino acid sequence can still have the inherent function. Thus, another specific example of the mutant CPT family protein is the following protein [A-2]:
[A-2] a protein having an amino acid sequence containing one or more amino acid substitutions, deletions, insertions, and/or additions relative to the amino acid sequence represented by SEQ ID NO:7, and having an enzyme activity that catalyzes a reaction of cis-chain elongation of an isoprenoid compound.

Here, in order to maintain the function of the mutant CPT family protein, it preferably has an amino acid sequence containing one or more, more preferably 1 to 53, still more preferably 1 to 40, further preferably 1 to 26, particularly preferably 1 to 13, most preferably 1 to 5, even most preferably 1 to 3 amino acid substitutions, deletions, insertions, and/or additions relative to the amino acid sequence of SEQ ID NO:7.

Conservative substitutions are preferred as examples of amino acid substitutions. Specific examples include substitutions within each of the following groups in the parentheses: (glycine, alanine), (valine, isoleucine, leucine), (aspartic acid, glutamic acid), (asparagine, glutamine), (serine, threonine), (lysine, arginine), and (phenylalanine, tyrosine).

It is also known that proteins with amino acid sequences having high sequence identity to the original amino acid sequence can also have similar functions. Thus, another specific example of the mutant CPT family protein is the following protein [A-3]:
[A-3] a protein having an amino acid sequence with at least 80% sequence identity to the amino acid sequence represented by SEQ ID NO:7, and having an enzyme activity that catalyzes a reaction of cis-chain elongation of an isoprenoid compound.

Here, in order to maintain the function of the mutant CPT family protein, the sequence identity to the amino acid sequence of SEQ ID NO:7 is preferably at least 85%, more preferably at least 90%, still more preferably at least 95%, particularly preferably at least 98%, most preferably at least 99%.

The sequence identity between amino acid sequences or nucleotide sequences may be determined using the algorithm BLAST [Pro. Natl. Acad. Sci. USA, 90, 5873 (1993), which is herein incorporated by reference] developed by Karlin and Altschul or FASTA [Methods Enzymol., 183, 63 (1990), which is herein incorporated by reference] .

Whether it is a protein having the above enzyme activity may be determined by, for example, conventional techniques, such as by expressing a target protein in a transformant produced by introducing a gene coding for the target protein into *Escherichia coli* or other host organisms, and determining the presence or absence of the function of the target protein by the corresponding activity measuring method.

Specific examples of the gene coding for the mutant CPT family protein include the following DNAs [B-1] and [B-2] :
[B-1] a DNA having the nucleotide sequence represented by SEQ ID NO:8; and
[B-2] a DNA which hybridizes under stringent conditions to a DNA having a nucleotide sequence complementary to the nucleotide sequence represented by SEQ ID NO:8, and which codes for a protein having an enzyme activity that catalyzes a reaction of cis-chain elongation of an isoprenoid compound.

Herein, the term "hybridize" means a process in which a DNA hybridizes to a DNA having a specific nucleotide sequence or a part of the DNA. Thus, the DNA having a specific nucleotide sequence or part of the DNA may have a nucleotide sequence long enough to be usable as a probe in Northern or Southern blot analysis or as an oligonucleotide primer in polymerase chain reaction (PCR) analysis. The DNA used as a probe may have a length of at least 100 bases, preferably at least 200 bases, more preferably at least 500 bases, but it may be a DNA of at least 10 bases, preferably at least 15 bases.

Techniques to perform DNA hybridization experiments are well known. The hybridization conditions under which experiments are carried out may be determined in accordance with, for example, Molecular Cloning, 2nd ed. and 3rd ed. (2001), Methods for General and Molecular Bacteriology, ASM Press (1994), Immunology methods manual, Academic press (Molecular), each of which is herein incorporated by reference, and many other standard textbooks.

The stringent conditions may include, for example, an overnight incubation at 42°C of a DNA-immobilized filter and a DNA probe in a solution containing 50% formamide, 5× SSC (750 mM sodium chloride, 75 mM sodium citrate), 50 mM sodium phosphate (pH 7.6), 5× Denhardt's solution, 10% dextran sulfate, and 20 µg/L denatured salmon sperm DNA, followed by washing the filter for example in a 0.2× SSC solution at approximately 65°C. Less stringent conditions may also be employed. Changes in stringency may be accomplished through the manipulation of formamide concentration (lower percentages of formamide result in lower stringency), salt concentrations, or temperature.
For example, low stringent conditions include an overnight incubation at 37°C in a solution containing 6× SSCE (20× SSCE: 3 mol/L sodium chloride, 0.2 mol/L sodium dihydrogen phosphate, 0.02 mol/L EDTA, pH 7.4), 0.5% SDS, 30% formamide, and 100 µg/L denatured salmon sperm DNA, followed by washing in a 1× SSC solution containing 0.1% SDS at 50°C. In addition, to achieve even lower stringency, washes performed following hybridization may be done at higher salt concentrations (e.g., 5× SSC) in the above-mentioned low stringent conditions.

Variations in the above conditions may be accomplished through the addition or substitution of blocking reagents used to suppress background in hybridization experiments. The addition of blocking reagents may require modification of the hybridization conditions for compatibility.

The DNA capable of hybridizing under stringent conditions as described above may have a nucleotide sequence with at least 80%, preferably at least 90%, more preferably at least 95%, still more preferably at least 98%, particularly preferably at least 99% sequence identity to the nucleotide sequence of SEQ ID NO:8 as calculated using a program such as BLAST or FASTA with the parameters mentioned above.

Whether the DNA which hybridizes to the above-mentioned DNA under stringent conditions codes for a protein having a predetermined enzyme activity may be determined by conventional techniques, such as by expressing a target protein in a transformant produced by introducing a gene coding for the target protein into *Escherichia coli* or other host organisms, and determining the presence or absence of the function of the target protein by the corresponding activity measuring method.

Moreover, conventional techniques may be employed to identify the amino acid sequence and nucleotide sequence of the protein. For example, total RNA is extracted from a growing plant, the mRNA is optionally purified, and a cDNA is synthesized by a reverse transcription reaction. Subsequently, degenerate primers are designed based on the amino acid sequence of a known protein corresponding to the target protein, a DNA fragment is partially amplified by RT-PCR, and the sequence is partially identified. Then, the full-length nucleotide sequence and amino acid sequence are identified by the RACE method, for example. The RACE method (rapid amplification of cDNA ends method) refers to a method in which, when the nucleotide sequence of a cDNA is partially known, PCR is performed based on the nucleotide sequence data of such a known region to clone the unknown region extending to the cDNA terminal. This method can clone full-length cDNA by PCR without preparing a cDNA library.

Here, the degenerate primers may each preferably be prepared from a plant-derived sequence having a highly similar sequence part to the target protein.

Moreover, if the nucleotide sequence coding for the protein is known, the full-length nucleotide sequence and amino acid sequence can be identified by designing a primer containing a start codon and a primer containing a stop codon using the known nucleotide sequence, followed by performing RT-PCR using a synthesized cDNA as a template.

### <Method for producing polyisoprenoid>

The method for producing a polyisoprenoid of the present invention is characterized by using the mutant cis-prenyltransferase (CPT) family protein of the present invention.

The method for producing a polyisoprenoid of the present invention may be any method using the mutant CPT family protein of the present invention. The method preferably includes binding the mutant CPT family protein of the present invention to membrane particles (preferably rubber particles) *in vitro.* Binding the mutant CPT family protein to membrane particles (preferably rubber particles) *in vitro* permits the synthesis of a polyisoprenoid in the membrane particles (preferably rubber particles), which enables the production of a higher molecular weight polyisoprenoid. This is because the produced polyisoprenoid will accumulate inside the membrane particles (preferably rubber particles), which enables the formation of a product having a longer chain length than that produced when the protein is not bound to membrane particles (preferably rubber particles).

Herein, the term "polyisoprenoid" is a collective term for polymers composed of isoprene units (C₅H₈), specifically natural products including a cis-1,4-polyisoprene or trans-1,4-polyisoprene as the basic carbon skeleton. Examples of the polyisoprenoid include polymers such as solanesol (C₄₅), undecaprenyl phosphate (Css), gutta percha, and rubber. The polyisoprenoid is preferably a cis-polyisoprenoid in which isoprene units are bound in a cis configuration. Moreover, herein, the term "isoprenoid" refers to a compound containing an isoprene unit (C₅H₈) and conceptually includes polyisoprenoids.

The membrane particles may be any particle having a membrane structure. Examples include biomembranes such as rubber particles and lipid droplets, and artificial membranes such as nanodiscs and liposomes. These may be used alone or in combinations of two or more. Rubber particles are preferred among these. The description below will discuss embodiments in which the membrane particles are rubber particles. The same may apply to other membrane particles.

Here, the production method of the present invention may include steps other than the binding step, and each step may be performed once or repeated multiple times.

Moreover, the amount of the mutant CPT family protein to be bound to the rubber particles is not limited.

Herein, the expression "the mutant CPT family protein binds to rubber particles" means, for example, that the mutant CPT family protein is fully or partially incorporated into the rubber particles or fully or partially inserted into the membrane structure of the rubber particles. It is not limited to these embodiments and also means embodiments in which the protein is localized on the surface or inside of the rubber particles. Moreover, the concept of binding to rubber particles also includes embodiments in which the mutant CPT family protein forms a complex with a protein bound to the rubber particles to be present in the form of the complex on the rubber particles. The same may apply to other membrane particles.

The origin of the rubber particles is not limited. For example, the rubber particles may be derived from the latex of a rubber-producing plant such as *Hevea brasiliensis, Taraxacum kok-saghyz, Parthenium argentatum,* or *Sonchus oleraceus.*

The particle size of the rubber particles is also not limited. Rubber particles having a predetermined particle size may be sorted out and used, or a mixture of rubber particles having different particle sizes may be used. Even when rubber particles having a predetermined particle size are sorted out and used, the rubber particles used may be either small rubber particles (SRP) having a small particle size or large rubber particles (LRP) having a large particle size.

Commonly used methods may be employed for sorting out the rubber particles having a predetermined particle size, including, for example, methods which involve centrifugation, preferably multistage centrifugation. An exemplary specific method includes centrifugation at 500-1500 × g, centrifugation at 1700-2500 × g, centrifugation at 7000-9000 × g, centrifugation at 15000-25000 × g, and centrifugation at 40000-60000 × g, carried out in said order. Here, the duration of each centrifugation is preferably 20 minutes or longer, more preferably 30 minutes or longer, still more preferably 40 minutes or longer, but is preferably 120 minutes or shorter, more preferably 90 minutes or shorter. Moreover, the temperature for each centrifugation is preferably 0 to 10°C, more preferably 2 to 8°C, particularly preferably 4°C.

In the binding step, a protein expressed from a gene coding for the mutant CPT family protein is bound to rubber particles *in vitro.*

The gene coding for the mutant CPT family protein is as described above.

Moreover, as the mutant CPT family protein is obtained by mutating the amino acid sequence of a N-terminal region of a CPT family protein not found on rubber particles to be identical or similar to the amino acid sequence of a N-terminal region of a CPT family protein found on rubber particles, when the origin of the CPT family protein found on rubber particles is the same as the origin of the rubber particles to be bound to the mutant CPT family protein, the origin of the amino acid sequence of the N-terminal region of the mutant CPT family protein is the same as the origin of the rubber particles to be bound to the mutant CPT family protein, so that the binding ability between the mutant CPT family protein and the rubber particles can be further enhanced.

For example, when rubber particles derived from *Hevea brasiliensis* are used, the mutant protein used is preferably the mutant CPT family protein in which the N-terminal region is substituted by a N-terminal region of a CPT family protein found on rubber particles derived from *Hevea brasiliensis* (HRT1). Or, when rubber particles derived from *Taraxacum kok-saghyz* are used, the mutant protein used is preferably the mutant CPT family protein in which the N-terminal region is substituted by a N-terminal region of a CPT family protein found on rubber particles derived from *Taraxacum kok-saghyz.*

Here, in the binding step, as long as the protein expressed from the gene coding for the mutant CPT family protein is bound to rubber particles *in vitro,* one or more additional proteins may also be bound thereto.

The origin of the additional proteins is not limited, but the additional proteins are preferably derived from a plant, more preferably a rubber-producing plant, still more preferably at least one selected from the group consisting of plants of the genera *Hevea, Sonchus, Taraxacum,* and *Parthenium.* In particular, they are further preferably derived from at least one plant selected from the group consisting of *Hevea brasiliensis, Sonchus oleraceus, Parthenium argentatum,* and *Taraxacum kok-saghyz,* particularly preferably from *Hevea brasiliensis.*

The additional proteins are not limited and may be any protein, but in view of the rubber synthesis ability of the rubber particles, they are each preferably a protein that is naturally found on rubber particles in a rubber-producing plant. Here, the protein found on rubber particles may be a protein that binds to a large part of the membrane surface of rubber particles, or a protein that binds to the membrane of rubber particles so as to be inserted thereinto, or a protein that forms a complex with a protein bound to the membrane to be present on the membrane surface.

Examples of the protein that is naturally found on rubber particles in a rubber-producing plant include HRT1-REF-bridging protein (HRBP), rubber elongation factor (REF), small rubber particle protein (SRPP), β-1,3-glucanase, and Hevein. HRT1-REF-bridging protein (HRBP) or rubber elongation factor (REF) is preferred among these, with REF being more preferred.

The binding step may be carried out using any means that can bind the mutant CPT family protein to rubber particles *in vitro,* such as, for example, by performing protein synthesis in the presence of both rubber particles and a cell-free protein synthesis solution containing an mRNA coding for the mutant CPT family protein to bind the mutant CPT family protein to the rubber particles.

The binding step preferably includes performing protein synthesis in the presence of both rubber particles and a cell-free protein synthesis solution containing an mRNA coding for the mutant CPT family protein to bind the mutant CPT family protein to the rubber particles, among other methods.

In other words, it is preferred to obtain rubber particles bound to the mutant CPT family protein by performing protein synthesis in the presence of both rubber particles and a cell-free protein synthesis solution containing an mRNA coding for the mutant CPT family protein, or, more specifically, using a mixture of rubber particles with a cell-free protein synthesis solution containing an mRNA coding for the mutant CPT family protein.

The protein synthesis performed in the presence of both rubber particles and a cell-free protein synthesis solution containing an mRNA coding for the mutant CPT family protein is namely the synthesis of the mutant CPT family protein by cell-free protein synthesis, which can synthesize the mutant CPT family protein maintaining its biological function (native state). The cell-free protein synthesis performed in the presence of rubber particles allows the synthesized mutant CPT family protein in the native state to bind to the rubber particles.

Here, the expression "performing protein synthesis in the presence of both the cell-free protein synthesis solution and rubber particles to bind the mutant CPT family protein to the rubber particles" means, for example, that each mutant CPT family protein synthesized by the protein synthesis is fully or partially incorporated into the rubber particles or fully or partially inserted into the membrane structure of the rubber particles. It is not limited to these embodiments and also means embodiments in which, for example, the protein is localized on the surface or inside of the rubber particles. Moreover, the concept of binding to rubber particles also includes embodiments in which the protein forms a complex with a protein bound to the rubber particles as described above to be present in the form of the complex on the rubber particles.

Each mRNA coding for the mutant CPT family protein serves as a translation template that can be translated to synthesize the mutant CPT family protein.

Each mRNA coding for the mutant CPT family protein may be prepared by any method as long as the prepared mRNA serves as a translation template that can be translated to synthesize the mutant CPT family protein.

As long as the cell-free protein synthesis solution contains the mRNA coding for the mutant CPT family protein, it may contain an mRNA coding for an additional protein.

The mRNA coding for an additional protein may be one that can be translated to express the additional protein. Here, the additional protein may be as described above.

In the binding step, cell-free protein synthesis of the mutant CPT family protein is preferably performed in the presence of rubber particles. This cell-free protein synthesis may be carried out using the cell-free protein synthesis solution in a similar manner to conventional methods. The cell-free protein synthesis system used may be a common cell-free protein synthesis means, examples of which include rapid translation system RTS500 (Roche Diagnostics); and wheat germ extracts prepared in accordance with Proc. Natl. Acad. Sci. USA, 97:559-564 (2000), JP 2000-236896 A, JP 2002-125693 A, and JP 2002-204689 A, each of which is herein incorporated by reference, or their cell-free protein synthesis systems (JP 2002-204689 A, Proc. Natl. Acad. Sci. USA, 99:14652-14657 (2002), each of which is herein incorporated by reference). Systems using germ extracts are preferred among these.

The source of the germ extracts is not limited. From the standpoint of translation efficiency, it is preferred to use a plant-derived germ extract for cell-free protein synthesis of a plant protein. It is particularly preferred to use a wheat-derived germ extract.

The method for preparing such a germ extract is not limited, and may be carried out conventionally, as described in, for example, JP 2005-218357 A, which is herein incorporated by reference.

The cell-free protein synthesis solution preferably further contains a cyclic nucleoside monophosphate derivative or a salt thereof (hereinafter, also referred to simply as "activity enhancer"). Protein synthesis activity can be further enhanced by the inclusion of the activity enhancer.

The cyclic nucleoside monophosphate derivative or salt thereof is not limited as long as it can enhance cell-free protein synthesis activity. Examples include adenosine-3',5'-cyclic monophosphoric acid and its salts; adenosine-3',5'-cyclic monophosphorothioic acid (Sp-isomer) and its salts; adenosine-3',5'-cyclic monophosphorothioic acid (Rp-isomer) and its salts; guanosine-3',5'-cyclic monophosphoric acid and its salts; guanosine-3',5'-cyclic monophosphorothioic acid (Sp-isomer) and its salts; guanosine-3',5'-cyclic monophosphorothioic acid (Rp-isomer) and its salts; 8-bromoadenosine-3',5'-cyclic monophosphoric acid (bromo-cAMP) and its salts; 8-(4-chlorophenylthio)adenosine-3',5'-cyclic monophosphoric acid (chlorophenylthio-cAMP) and its salts; 5,6-dichloro-1-β-D-ribofuranosylbenzimidazole adenosine-3',5'-cyclic monophosphoric acid (dichlororibofuranosylbenzimidazole cAMP) and its salts; adenosine-2',5'-cyclic monophosphoric acid and its salts; adenosine-2',5'-cyclic monophosphorothioic acid (Sp-isomer) and its salts; adenosine-2',5'-cyclic monophosphorothioic acid (Rp-isomer) and its salts; guanosine-2',5'-cyclic monophosphoric acid and its salts; guanosine-2',5'-cyclic monophosphorothioic acid (Sp-isomer) and its salts; and guanosine-2',5'-cyclic monophosphorothioic acid (Rp-isomer) and its salts.

The base that forms a salt with the cyclic nucleoside monophosphate derivative is not limited as long as it is biochemically acceptable and forms a salt with the derivative. Preferred are, for example, alkali metal atoms such as sodium or potassium, and organic bases such as tris-hydroxyaminomethane, among others.

Among these activity enhancers, adenosine-3',5'-cyclic monophosphoric acid or adenosine-3',5'-cyclic monophosphate sodium salt is particularly preferred. Moreover, these activity enhancers may be used alone or in combinations of two or more.

The activity enhancer may be added to the cell-free protein synthesis solution in advance, but if the activity enhancer is unstable in the solution, it is preferably added during the protein synthesis reaction performed in the presence of both the cell-free protein synthesis solution and rubber particles.

The amount of the activity enhancer(s) added is not limited as long as it is at a concentration that can activate (increase) the protein synthesis reaction in the cell-free protein synthesis solution. Specifically, the final concentration in the reaction system may usually be at least 0.1 millimoles/liter. The lower limit of the concentration is preferably 0.2 millimoles/liter, more preferably 0.4 millimoles/liter, particularly preferably 0.8 millimoles/liter, while the upper limit of the concentration is preferably 24 millimoles/liter, more preferably 6.4 millimoles/liter, particularly preferably 3.2 millimoles/liter.

The temperature of the cell-free protein synthesis solution at which the activity enhancer is added to the cell-free protein synthesis solution is not limited, but is preferably 0 to 30°C, more preferably 10 to 26°C.

In addition to the mRNA (translation template) coding for the mutant CPT family protein, the cell-free protein synthesis solution also contains ATP, GTP, creatine phosphate, creatine kinase, L-amino acids, potassium ions, magnesium ions, and other components required for protein synthesis, and optionally an activity enhancer. The use of such a cell-free protein synthesis solution can provide a cell-free protein synthesis reaction system.

Here, since the germ extract prepared as described in JP 2005-218357 A above contains tRNA in an amount necessary for protein synthesis reaction, addition of separately prepared tRNA is not required when the germ extract prepared as above is used in the cell-free protein synthesis solution. In other words, tRNA may be added to the cell-free protein synthesis solution, if necessary.

The binding step preferably includes performing protein synthesis in the presence of both rubber particles and a cell-free protein synthesis solution containing an mRNA coding for the mutant CPT family protein. Specifically, this can be accomplished by adding rubber particles to the cell-free protein synthesis solution at a suitable time either before or after the protein synthesis, preferably before the protein synthesis.

Moreover, the concentration of rubber particles present with the cell-free protein synthesis solution is preferably 5 to 50 g/L. In other words, 5 to 50 g of rubber particles are preferably present in 1 L of the cell-free protein synthesis solution. If the concentration of rubber particles present with the cell-free protein synthesis solution is less than 5 g/L, a rubber layer may not be formed by separation treatment (e.g., ultracentrifugation) for collecting the rubber particles bound to the synthesized mutant CPT family protein, and therefore it may be difficult to collect the rubber particles bound to the synthesized mutant CPT family protein. Moreover, if the concentration of rubber particles present with the cell-free protein synthesis solution exceeds 50 g/L, the rubber particles may aggregate, so that the synthesized mutant CPT family protein may fail to bind well to the rubber particles. The concentration of rubber particles is more preferably 10 to 40 g/L, still more preferably 15 to 35 g/L, particularly preferably 15 to 30 g/L.

Moreover, in the protein synthesis in the presence of both rubber particles and the cell-free protein synthesis solution, additional rubber particles may be appropriately added as the reaction progresses. The cell-free protein synthesis solution and rubber particles are preferably allowed to be present together during the period when the cell-free protein synthesis system is active, such as 3 to 48 hours, preferably 3 to 30 hours, more preferably 3 to 24 hours after the addition of rubber particles to the cell-free protein synthesis solution.

The rubber particles do not have to be subjected to any treatment, e.g., pretreatment, before use in the binding step, preferably before being combined with the cell-free protein synthesis solution. However, the proteins present on the rubber particles may be removed to some extent from the rubber particles with a surfactant beforehand to increase the proportion of the mutant CPT family protein desired to be bound. Then, the rubber particles after the removal preferably have a residual rubber synthesis activity that is at least 50% of that before the removal.

The surfactant is not limited, and examples include nonionic surfactants and amphoteric surfactants. Nonionic or amphoteric surfactants, among others, are suitable because they have only a little denaturing effect on the proteins on the membrane, and amphoteric surfactants are especially suitable. Thus, in another suitable embodiment of the first invention, the surfactant is an amphoteric surfactant.

These surfactants may be used alone or in combinations of two or more.

Examples of the nonionic surfactants include polyoxyalkylene ether nonionic surfactants, polyoxyalkylene ester nonionic surfactants, polyhydric alcohol fatty acid ester nonionic surfactants, sugar fatty acid ester nonionic surfactants, alkyl polyglycoside nonionic surfactants, and polyoxyalkylene polyglucoside nonionic surfactants; and polyoxyalkylene alkylamines and alkyl alkanolamides.

Polyoxyalkylene ether or polyhydric alcohol fatty acid ester nonionic surfactants are preferred among these.

Examples of the polyoxyalkylene ether nonionic surfactants include polyoxyalkylene alkyl ethers, polyoxyalkylene alkylphenyl ethers, polyoxyalkylene polyol alkyl ethers, and polyoxyalkylene mono-, di- or tristyrylphenyl ethers. Among these, polyoxyalkylene alkylphenyl ethers are suitable. Here, the polyol is preferably a C2-C12 polyhydric alcohol, such as ethylene glycol, propylene glycol, glycerin, sorbitol, glucose, sucrose, pentaerythritol, or sorbitan.

Examples of the polyoxyalkylene ester nonionic surfactants include polyoxyalkylene fatty acid esters and polyoxyalkylene alkyl rosin acid esters.

Examples of the polyhydric alcohol fatty acid ester nonionic surfactants include fatty acid esters of C2-C12 polyhydric alcohols and fatty acid esters of polyoxyalkylene polyhydric alcohols. More specific examples include sorbitol fatty acid esters, sorbitan fatty acid esters, glycerin fatty acid esters, polyglycerin fatty acid esters, and pentaerythritol fatty acid esters, as well as polyalkylene oxide adducts of the foregoing such as polyoxyalkylene sorbitan fatty acid esters and polyoxyalkylene glycerin fatty acid esters. Among these, sorbitan fatty acid esters are suitable.

Examples of the sugar fatty acid ester nonionic surfactants include fatty acid esters of sucrose, glucose, maltose, fructose, and polysaccharides, as well as polyalkylene oxide adducts of the foregoing.

Examples of the alkyl polyglycoside nonionic surfactants include those having, for example, glucose, maltose, fructose, or sucrose as the glycoside, such as alkyl glucosides, alkyl polyglucosides, polyoxyalkylene alkyl glucosides, and polyoxyalkylene alkyl polyglucosides, as well as fatty acid esters of the foregoing. Polyalkylene oxide adducts of any of the foregoing may also be used.

Examples of the alkyl groups in these nonionic surfactants include C4-C30 linear or branched, saturated or unsaturated alkyl groups. Moreover, the polyoxyalkylene groups may have C2-C4 alkylene groups, and may have about 1 to 50 moles of added ethylene oxide, for example. Moreover, examples of the fatty acids include C4-C30 linear or branched, saturated or unsaturated fatty acids.

Among the nonionic surfactants, polyoxyethyleneethylene (10) octylphenyl ether (Triton X-100) or sorbitan monolaurate (Span 20) is particularly preferred for their ability to moderately remove membrane-associated proteins while keeping the membrane of rubber particles stable and, further, having only a little denaturing effect on proteins.

Examples of the amphoteric surfactants include zwitterionic surfactants such as quaternary ammonium base group/sulfonic acid group (-SO₃H) surfactants, (watersoluble) quaternary ammonium base group/phosphoric acid group surfactants, (water-insoluble) quaternary ammonium base group/ phosphoric acid group surfactants, and quaternary ammonium base group/carboxyl group surfactants. Here, the acid groups may be salts.

In particular, such a zwitterionic surfactant preferably has both positive and negative charges in a molecule, and the acid dissociation constant (pKa) of the acid group is preferably 5 or less, more preferably 4 or less, still more preferably 3 or less.

Specific examples of the amphoteric surfactants include ammonium sulfobetaines such as 3-[(3-cholamidopropyl)dimethylammonio]-2-hydroxy-1-propanesulfonate (CHAPSO), 3-[(3-cholamidopropyl)-dimethylammonio]-propanesulfonate (CHAPS), N,N-bis(3-D-gluconamidopropyl)-cholamide, n-octadecyl-N,N'-dimethyl-3-ammonio-1-propanesulfonate, n-decyl-N,N'-dimethyl-3-ammonio-1-propanesulfonate, n-dodecyl-N,N'-dimethyl-3-ammonio-1-propanesulfonate, n-tetradecyl-N,N'-dimethyl-3-ammonio-1-propanesulfonate (Zwittergent^{®} 3-14), and n-hexadecyl-N,N'-dimethyl-3-ammonio-1-propanesulfonate; phosphocholines such as n-octylphosphocholine, n-nonylphosphocholine, n-decylphosphocholine, n-dodecylphosphocholine, n-tetradecylphosphocholine, and n-hexadecylphosphocholine; and phosphatidylcholines such as dilauroyl phosphatidylcholine, dimyristoyl phosphatidylcholine, dipalmitoyl phosphatidylcholine, distearoyl phosphatidylcholine, dioleoyl phosphatidylcholine, and dilinoleoyl phosphatidylcholine. Among these, 3-[(3-cholamidopropyl)dimethylammonio]-propanesulfonate (CHAPS) is particularly preferred for its ability to moderately remove proteins while keeping the membrane of rubber particles stable.

The surfactant concentration for the treatment is preferably within three times the critical micelle concentration (CMC) of the surfactant used. The membrane stability of the rubber particles may be reduced if they are treated with the surfactant at a concentration exceeding three times the critical micelle concentration. The concentration is more preferably within 2.5 times, still more preferably within 2.0 times the CMC. Moreover, the lower limit is preferably at least 0.05 times, more preferably at least 0.1 times, still more preferably at least 0.3 times the CMC.

Examples of reaction systems or apparatuses for protein synthesis that may be used in the cell-free protein synthesis include a batch method (Pratt, J.M. et al., Transcription and Translation, Hames, 179-209, B.D. & Higgins, S.J., eds, IRL Press, Oxford (1984), which is herein incorporated by reference), a continuous cell-free protein synthesis system in which amino acids, energy sources, and other components are supplied continuously to the reaction system (Spirin, A.S. et al., Science, 242, 1162-1164 (1988), which is herein incorporated by reference), a dialysis method (Kigawa et al., 21st Annual Meeting of the Molecular Biology Society of Japan, WID 6, which is herein incorporated by reference), and an overlay method (instruction manual of PROTEIOS^{™} wheat germ cell-free protein synthesis core kit, Toyobo Co., Ltd., which is herein incorporated by reference). Other methods may include supplying template RNA, amino acids, energy sources, and other components, if necessary, to the protein synthesis reaction system, and discharging the synthesis product or decomposition product as required.

The dialysis method is preferred among these. The reason for this is as follows. The overlay method has the advantage of easy operation, but unfortunately rubber particles disperse in the reaction solution and thus are difficult to efficiently bind to the synthesized mutant CPT family protein. In contrast, in the dialysis method, since the amino acids used as the raw materials of the mutant CPT family protein to be synthesized can pass through the dialysis membrane while rubber particles cannot pass therethrough, it is possible to prevent dispersal of rubber particles and thus to efficiently bind the synthesized mutant CPT family protein to rubber particles.

Here, the dialysis method refers to a method in which protein synthesis is performed using an apparatus in which the synthesis reaction solution for protein synthesis used in the cell-free protein synthesis is used as an internal dialysis solution and is separated from an external dialysis solution by a dialysis membrane capable of mass transfer. Specifically, for example, the synthesis reaction solution excluding the translation template is optionally pre-incubated for an appropriate amount of time, to which is then added the translation template and the mixture is put in an appropriate dialysis container as the internal reaction solution. Examples of the dialysis container include containers with a dialysis membrane attached to the bottom (e.g., Dialysis Cup 12,000 available from Daiichi Kagaku) and dialysis tubes (e.g., 12,000 available from Sanko Junyaku Co., Ltd.). The dialysis membrane used may have a molecular weight cutoff of 10,000 daltons or more, preferably about 12,000 daltons.

The external dialysis solution used may be a buffer containing amino acids. Dialysis efficiency can be increased by replacing the external dialysis solution with a fresh one when the reaction speed declines. The reaction temperature and time may be selected appropriately according to the protein synthesis system used. For example, in the case of a system using a wheat-derived germ extract, the reaction may be carried out usually at 10 to 40°C, preferably 18 to 30°C, more preferably 20 to 26°C, for 10 minutes to 48 hours, preferably for 10 minutes to 30 hours, more preferably for 10 minutes to 24 hours.

Moreover, since the mRNA coding for the mutant CPT family protein contained in the cell-free protein synthesis solution is easily broken down, the mRNA may be additionally added as appropriate during the protein synthesis reaction to make the protein synthesis more efficient. Here, the addition time, the number of additions, the addition amount, and other conditions of the mRNA are not limited and may be selected appropriately.

In the production method of the first invention, the step of binding a protein expressed from a gene coding for the mutant CPT family protein to rubber particles *in vitro* may optionally be followed by collecting the rubber particles.

The rubber particle collection step may be carried out by any method that can collect the rubber particles. It may be carried out by conventional methods for collecting rubber particles. Specific examples include methods using centrifugation. When the rubber particles are collected by the centrifugation methods, the centrifugal force, centrifugation time, and centrifugation temperature may be selected appropriately so as to be able to collect the rubber particles. For example, the centrifugal force during the centrifugation is preferably 15000 × g or more, more preferably 20000 × g or more, still more preferably 25000 × g or more. However, since increasing the centrifugal force too much is not expected to produce a correspondingly high separation effect, the upper limit of the centrifugal force is preferably 50000 × g or less, more preferably 45000 × g or less. The centrifugation time is preferably at least 20 minutes, more preferably at least 30 minutes, still more preferably at least 40 minutes. However, since increasing the centrifugation time too much is not expected to produce a correspondingly high separation effect, the upper limit of the centrifugation time is preferably 120 minutes or less, more preferably 90 minutes or less.

Moreover, from the standpoint of maintaining the activity of the mutant CPT family protein bound to rubber particles, the centrifugation temperature is preferably 0 to 10°C, more preferably 2 to 8°C, particularly preferably 4°C.

For example, when the cell-free protein synthesis is performed, the centrifugation may be performed to separate the rubber particles and the cell-free protein synthesis solution into the upper and lower layers, respectively.
The cell-free protein synthesis solution as the lower layer may then be removed to collect the rubber particles bound to the mutant CPT family protein. The collected rubber particles may be re-suspended in an appropriate buffer having a neutral pH for storage.

Here, the rubber particles collected by the rubber particle collection step can be used in the same way as usual natural rubber without the need for further special treatment.

Further, the polyisoprenoid produced by the method for producing a polyisoprenoid of the first invention may be recovered by subjecting the rubber particles to the solidification step described below.

The solidification step may be carried out by any solidification method, such as by adding the rubber particles to a solvent that does not dissolve polyisoprenoids, such as ethanol, methanol, or acetone, or by adding an acid to the rubber particles. Rubber (a type of polyisoprenoid) can be recovered as solids from the rubber particles by the solidification step. The obtained rubber may be dried if necessary before use.

Thus, according to the first invention, by binding a protein expressed from a gene coding for the mutant CPT family protein to rubber particles *in vitro,* it is possible to synthesize a polyisoprenoid in the rubber particles, and therefore it is possible to efficiently produce a high molecular weight polyisoprenoid in a reaction vessel (e.g., a test tube or industrial plant).

### (Method for producing rubber product)

The method for producing a rubber product of the first invention includes: producing a polyisoprenoid by the method for producing a polyisoprenoid of the first invention; kneading the polyisoprenoid with an additive to obtain a kneaded mixture; forming a raw rubber product from the kneaded mixture; and vulcanizing the raw rubber product.

The rubber product may be any rubber product that can be produced from rubber, preferably natural rubber. Examples include pneumatic tires, rubber rollers, rubber fenders, gloves, and medical rubber tubes.

When the rubber product is a pneumatic tire, or in other words, when the method for producing a rubber product of the first invention is the method for producing a pneumatic tire of the first invention, the raw rubber product forming step corresponds to the step of building a green tire from the kneaded mixture, and the vulcanization step corresponds to the step of vulcanizing the green tire. Thus, the method for producing a pneumatic tire of the first invention includes: producing a polyisoprenoid by the method for producing a polyisoprenoid; kneading the polyisoprenoid with an additive to obtain a kneaded mixture; building a green tire from the kneaded mixture; and vulcanizing the green tire.

### <Kneading step>

In the kneading step, the polyisoprenoid produced by the method for producing a polyisoprenoid is kneaded with an additive to obtain a kneaded mixture.

Any additive may be used including those used in the production of rubber products. For example, when the rubber product is a pneumatic tire, examples of the additive include rubber components other than the polyisoprenoid, reinforcing fillers such as carbon black, silica, calcium carbonate, alumina, clay, and talc, silane coupling agents, zinc oxide, stearic acid, processing aids, various antioxidants, softeners such as oils, waxes, vulcanizing agents such as sulfur, and vulcanization accelerators.

The kneading in the kneading step may be carried out using a rubber kneading machine such as an open roll mill, a Banbury mixer, or an internal mixer.

### <Raw rubber product forming step (green tire building step in the case of tire)>

In the raw rubber product forming step, a raw rubber product (green tire in the case of tire) is formed from the kneaded mixture obtained in the kneading step.

The method for forming a raw rubber product is not limited, and methods used to form raw rubber products may be used appropriately. For example, when the rubber product is a pneumatic tire, the kneaded mixture obtained in the kneading step may be extruded into the shape of a tire component and then assembled with other tire components in a usual manner on a tire building machine to build a green tire (unvulcanized tire).

### <Vulcanization step>

In the vulcanization step, the raw rubber product obtained in the raw rubber product forming step is vulcanized to produce a rubber product.

The method for vulcanizing the raw rubber product is not limited, and methods used to vulcanize raw rubber products may be used appropriately. For example, when the rubber product is a pneumatic tire, the green tire (unvulcanized tire) obtained in the raw rubber product forming step may be vulcanized by heating and pressing in a vulcanizer to produce a pneumatic tire.

### (Second Invention)

### (Vector)

The vector of the second invention contains a gene coding for the mutant cis-prenyltransferase (CPT) family protein of the present invention. When this vector is introduced into a plant for transformation, the gene coding for the mutant CPT family protein in the vector will be expressed to enable the production of a higher molecular weight polyisoprenoid in the plant as compared to before the gene recombination.

The vector preferably contains a promoter for use in protein expression in a plant and a gene coding for the mutant CPT family protein, functionally linked to the promoter. In this case, the gene coding for the mutant CPT family protein can be more suitably expressed in a plant.

Herein, the expression "a gene is functionally linked to a promoter" means that the gene sequence is linked downstream of the promoter so that it is controlled by the promoter.

The promoter for use in protein expression in a plant may be any one capable of functioning in plant cells. Examples include a cauliflower mosaic virus (CaMV) 35S promoter, a rice actin 1 promoter, a nopaline synthase gene promoter, a tobacco mosaic virus 35S promoter, a rice-derived actin gene promoter, and a ubiquitin promoter.

The vector more preferably contains a promoter having a promoter activity that drives laticifer-specific gene expression and a gene coding for the mutant CPT family protein, functionally linked to the promoter. When this vector is introduced into a plant for transformation, the gene coding for the mutant CPT family protein in the vector will be laticifer-specifically expressed to more suitably enable the production of a higher molecular weight polyisoprenoid in the plant as compared to before the gene recombination.

Herein, the expression "promoter has a promoter activity that drives laticifer-specific gene expression" means that the promoter has activity to control gene expression to cause a desired gene to be expressed specifically in laticifers when the desired gene is functionally linked to the promoter and introduced into a plant. Here, the term "laticifer-specific gene expression" means that the gene is expressed substantially exclusively in laticifers with no or little expression of the gene in the sites other than laticifers in a plant.

For example, the vector of the second invention can be prepared by inserting the nucleotide sequence of a promoter having a promoter activity that drives laticifer-specific gene expression and the nucleotide sequence of a gene coding for the mutant CPT family protein into a vector commonly known as a plant transformation vector by conventional techniques. Examples of vectors that can be used to prepare the vector of the second invention include pBI vectors, binary vectors such as pGA482, pGAH, and pBIG, intermediate plasmids such as pLGV23Neo, pNCAT, and pMON200, and pH35GS containing GATEWAY cassette.

As long as the vector of the second invention contains the nucleotide sequence of a gene coding for the mutant CPT family protein, it may contain additional nucleotide sequences in addition to the nucleotide sequence of a promoter. In addition to these nucleotide sequences, the vector usually contains vector-derived sequences as well as other sequences such as a restriction enzyme recognition sequence, a spacer sequence, a marker gene sequence, and a reporter gene sequence.

Examples of the marker gene include drug-resistant genes such as a kanamycin-resistant gene, a hygromycin-resistant gene, and a bleomycin-resistant gene. Moreover, the reporter gene is intended to be introduced to determine the expression site in a plant, and examples include a luciferase gene, a β-glucuronidase (GUS) gene, a green fluorescent protein (GFP), and a red fluorescent protein (RFP) .

The gene coding for the mutant CPT family protein is as described above for the first invention.

The promoter having a promoter activity that drives laticifer-specific gene expression is preferably at least one selected from the group consisting of a promoter of a gene coding for rubber elongation factor (REF), a promoter of a gene coding for small rubber particle protein (SRPP), a promoter of a gene coding for Hevein 2.1 (HEV2.1), and a promoter of a gene coding for MYC1 transcription factor (MYC1).

Herein, the term "rubber elongation factor (REF)" refers to a rubber particle-associated protein that binds to rubber particles in the latex of rubber-producing plants such as *Hevea brasiliensis,* and contributes to stabilization of the rubber particles.

The term "small rubber particle protein (SRPP)" refers to a rubber particle-associated protein that binds to rubber particles in the latex of rubber-producing plants such as *Hevea brasiliensis.*

The term "Hevein 2.1 (HEV2.1)" refers to a protein that is highly expressed in the laticifer cells of rubber-producing plants such as *Hevea brasiliensis.* This protein is involved in coagulation of rubber particles and has antifungal activity.

Moreover, the term "MYC1 transcription factor (MYC1)" refers to a transcription factor that is highly expressed in the latex of rubber-producing plants such as *Hevea brasiliensis* and participates in jasmonic acid signaling. Here, the term "transcription factor" means a protein having activity to increase or decrease, preferably increase, gene transcription. In other words, MYC1 herein is a protein having activity (transcription factor activity) to increase or decrease, preferably increase, the transcription of a gene coding for at least one protein among the proteins involved in jasmonic acid signaling.

The vector of the second invention (which contains a gene coding for the mutant CPT family protein) may be introduced into a plant to produce a transgenic plant transformed to express the mutant CPT family protein involved in polyisoprenoid biosynthesis. Moreover, in the transgenic plant in which the mutant CPT family protein involved in polyisoprenoid biosynthesis is expressed, a certain function (e.g., enzyme activity) possessed by the protein is newly enhanced in the plant transfected with the vector of the second invention. As a result, the production of a higher molecular weight polyisoprenoid can be more suitably enabled in the plant as compared to before the gene recombination.

The method for preparing the transgenic plant is explained briefly below, though such a transgenic plant can be prepared by conventional methods.

The plant into which the vector of the second invention is to be introduced to produce the transgenic plant is not limited, but is preferably a rubber-producing plant, among others. This is because the production of a high molecular weight polyisoprenoid can be expected particularly when the mutant CPT family protein is expressed in a plant capable of biosynthesizing a polyisoprenoid. In particular, the plant is preferably a plant of the genus *Hevea* or *Taraxacum,* more preferably *Hevea brasiliensis* or *Taraxacum kok-saghyz.*

The vector of the second invention may be introduced into a plant (including plant cells, such as callus, cultured cells, spheroplasts, and protoplasts) by any method that can introduce DNA into plant cells. Examples include methods using *Agrobacterium* (JP S59-140885 A, JP S60-70080 A, WO94/00977, each of which is herein incorporated by reference), electroporation (JP S60-251887 A, which is herein incorporated by reference), and methods using particle guns (gene guns) (JP 2606856 B, JP 2517813 B, each of which is herein incorporated by reference). Among these, it is preferred to use a method using *Agrobacterium* (Agrobacterium method) to introduce the vector of the second invention into a plant to produce a transgenic plant (transgenic plant cells).

Furthermore, the vector of the second invention may also be introduced into, for example, an organism (e.g., a microorganism, yeast, animal cell, or insect cell) or a part thereof, an organ, a tissue, a cultured cell, a spheroplast, or a protoplast by any of the above or other DNA introduction methods to produce a cis-isoprenoid or polyisoprenoid.

The transgenic plant (transgenic plant cells) can be produced by the above or other methods. Here, the term "transgenic plant" conceptually includes not only transgenic plant cells produced by the above methods, but also all of their progeny or clones and even progeny plants obtained by subculturing the foregoing. Once obtaining transgenic plant cells into which the vector of the second invention has been introduced, progeny or clones can be produced from the transgenic plant cells by sexual or asexual reproduction, tissue culture, cell culture, cell fusion, or other techniques. Moreover, the transgenic plant cells, or their progeny or clones may be used to obtain reproductive materials (e.g., seeds, fruits, cuttings, stem tubers, root tubers, shoots, adventitious buds, adventitious embryos, callus, protoplasts), which may then be used to produce the transgenic plant on a large scale.

Techniques to regenerate plants (transgenic plants) from transgenic plant cells are already known; for example, Doi et al. disclose techniques for eucalyptus (Japanese Patent Application No. H11-127025, which is herein incorporated by reference), Fujimura et al. disclose techniques for rice (Fujimura et al., (1995), Plant Tissue Culture Lett., vol. 2: p. 74-, which is herein incorporated by reference), Shillito et al. disclose techniques for corn (Shillito et al., (1989), Bio/Technology, vol. 7: p. 581-, which is herein incorporated by reference), Visser et al. disclose techniques for potato (Visser et al., (1989), Theor. Appl. Genet., vol. 78: p. 589-, which is herein incorporated by reference), and Akama et al. disclose techniques for *Arabidopsis thaliana* (Akama et al., (1992), Plant Cell Rep., vol. 12: p. 7-, which is herein incorporated by reference). A person skilled in the art can regenerate plants from the transgenic plant cells with reference to these documents.

Whether a target protein gene is expressed in a regenerated plant may be determined by well-known methods. For example, Western blot analysis may be used to assess the expression of a target protein.

Seeds can be obtained from the transgenic plant, for example, as follows: The transgenic plant is rooted in an appropriate medium and transplanted to water-containing soil in a pot. The plant is grown under proper cultivation conditions to finally produce seeds, which are then collected. Moreover, plants can be grown from seeds, for example, as follows: Seeds obtained from the transgenic plant as described above are sown in water-containing soil and grown under proper cultivation conditions into plants.

According to the second invention, by introducing the vector of the second invention into a plant, the gene coding for the mutant CPT family protein, which is involved in polyisoprenoid biosynthesis, in the vector will be expressed to enable the production of a higher molecular weight polyisoprenoid in the plant as compared to before the gene recombination. Specifically, a high molecular weight cis-polyisoprenoid or polyisoprenoid can be produced by culturing, for example, transgenic plant cells produced as described above, callus obtained from the transgenic plant cells, or cells redifferentiated from the callus in an appropriate medium, or by growing, for example, transgenic plants regenerated from the transgenic plant cells, or plants grown from seeds obtained from these transgenic plants under proper cultivation conditions.

### (Method for producing rubber product)

The method for producing a rubber product of the second invention includes: producing a polyisoprenoid using a transgenic plant produced by introducing the vector of the second invention into a plant; kneading the polyisoprenoid with an additive to obtain a kneaded mixture; forming a raw rubber product from the kneaded mixture; and vulcanizing the raw rubber product.

The rubber product is as described above for the first invention.

When the rubber product is a pneumatic tire, or in other words, when the method for producing a rubber product of the second invention is the method for producing a pneumatic tire of the second invention, the raw rubber product forming step corresponds to the step of building a green tire from the kneaded mixture, and the vulcanization step corresponds to the step of vulcanizing the green tire. Thus, the method for producing a pneumatic tire of the second invention includes: producing a polyisoprenoid using a transgenic plant produced by introducing the vector of the second invention into a plant; kneading the polyisoprenoid with an additive to obtain a kneaded mixture; building a green tire from the kneaded mixture; and vulcanizing the green tire.

### <Kneading step>

In the kneading step, the polyisoprenoid produced by the transgenic plant produced by introducing the vector of the second invention into a plant is kneaded with an additive to obtain a kneaded mixture.

The polyisoprenoid produced by the transgenic plant produced by introducing the vector of the second invention into a plant can be collected by harvesting latex from the transgenic plant, and subjecting the latex to the solidification step described below.

Here, the method for harvesting latex from the transgenic plant is not limited, and conventional methods may be used. For example, latex may be harvested by collecting the emulsion oozing out from the cuts in the trunk of the plant (tapping), or the emulsion oozing out from the cut roots or other parts of the transgenic plant, or by crushing the cut tissue followed by extraction with an organic solvent.

### <Solidification step>

The harvested latex is subjected to a solidification step. The solidification may be carried out by any method, such as by adding the latex to a solvent that does not dissolve polyisoprenoids, such as ethanol, methanol, or acetone, or by adding an acid to the latex. Rubber (a type of polyisoprenoid) can be recovered as solids from the latex by the solidification step. The obtained rubber may be dried if necessary before use.

Any additive may be used including additives used in the production of rubber products. For example, when the rubber product is a pneumatic tire, examples of the additive include rubber components other than the rubber obtained from the latex, reinforcing fillers such as carbon black, silica, calcium carbonate, alumina, clay, and talc, silane coupling agents, zinc oxide, stearic acid, processing aids, various antioxidants, softeners such as oils, waxes, vulcanizing agents such as sulfur, and vulcanization accelerators.

The kneading in the kneading step may be carried out using a rubber kneading machine such as an open roll mill, a Banbury mixer, or an internal mixer.

### <Raw rubber product forming step (green tire building step in the case of tire)>

The raw rubber product forming step is as described above for the first invention.

### <Vulcanization step>

The vulcanization step is as described above for the first invention.

### EXAMPLES

The present invention is specifically described with reference to examples, but the present invention is not limited to the examples.

A summary of the example and comparative example will be described first.

Example 1: Assay on rubber particles using AtCPT5-N-terminal HRT1

Comparative Example 1: Assay on rubber particles using AtCPT5

In Example 1 and Comparative Example 1, the chain lengths of the products produced with a N-terminal mutant and a normal type of CPT incapable of interacting with a NgBR family protein were compared. Here, AtCPT5 corresponds to a CPT family protein not found on rubber particles, and HRT1 corresponds to a CPT family protein found on rubber particles.

### (Acquisition of each gene)

### (1) HRT1 gene

### [Extraction of total RNA from Hevea latex]

Total RNA was extracted from the latex of *Hevea brasiliensis* by the hot phenol method. To 6 mL of the latex were added 6 mL of a 100 mM sodium acetate buffer and 1 mL of a 10% SDS solution, followed by addition of 12 mL of water-saturated phenol pre-heated at 65°C. The mixture was incubated for five minutes at 65°C, agitated in a vortex mixer, and centrifuged at 7000 rpm for 10 minutes at room temperature. After the centrifugation, the supernatant was transferred to a new tube, 12 mL of a phenol:chloroform (1:1) solution was added, and they were agitated by shaking for two minutes. After the agitation, the resulting mixture was centrifuged again at 7000 rpm for 10 minutes at room temperature. Then, the supernatant was transferred to a new tube, 12 mL of a chloroform:isoamyl alcohol (24:1) solution was added, and they were agitated by shaking for two minutes. After the agitation, the resulting mixture was centrifuged again at 7000 rpm for 10 minutes at room temperature. Then, the supernatant was transferred to a new tube, 1.2 mL of a 3M sodium acetate solution and 13 mL of isopropanol were added, and they were agitated in a vortex mixer. The resulting mixture was incubated for 30 minutes at -20°C to precipitate total RNA. The incubated mixture was centrifuged at 15000 rpm for 10 minutes at 4°C, and the supernatant was removed to collect a precipitate of total RNA. The collected total RNA was washed twice with 70% ethanol and then dissolved in RNase-free water.

### [Synthesis of cDNA from total RNA]

cDNA was synthesized from the collected total RNA. The cDNA synthesis was carried out using a PrimeScript II 1st strand cDNA synthesis kit (Takara) in accordance with the manual.

### [Acquisition of HRT1 gene from cDNA]

The prepared 1st strand cDNA was used as a template to obtain a HRT1 gene. PCR was performed using a KOD-plus-Neo (Toyobo) in accordance with the manual. The PCR reaction involved 35 cycles with each cycle consisting of 10 seconds at 98°C, 30 seconds at 58°C, and 1 minute at 68°C.

The HRT1 gene was obtained using the following primers.
Primer 1: 5'-tttggatccgatggaattatacaacggtgagagg-3'
Primer 2: 5'-tttgcggccgcttattttaagtattccttatgtttctcc-3'

A HRT1 gene was prepared as described above. The gene was sequenced to identify the full-length nucleotide sequence and amino acid sequence.

### [Acquisition of REF gene from cDNA]

The following primers were used.
Primer 3: 5'-tttctcgagatggctgaagacgaagac-3'
Primer 4: 5'-tttggatcctcaattctctccataaaac-3'

A REF gene was prepared as described above. The gene was sequenced to identify the full-length nucleotide sequence and amino acid sequence (SEQ ID NO:9 and SEQ ID NO:10) .

### [Vector construction]

The obtained DNA fragments were subjected to dA addition and then inserted into a pGEM-T Easy vector using a pGEM-T Easy Vector System (Promega) to prepare pGEM-HRT1 and pGEM-REF.

### [Transformation of Escherichia coli]

*Escherichia coli* DH5α was transformed with the prepared vectors, and the transformant was cultured on LB agar medium containing ampicillin and X-gal. Then, *Escherichia coli* cells carrying the introduced target gene were selected by blue/white screening.

### [Plasmid extraction]

The *Escherichia coli* cells transformed with the plasmid containing the target gene were cultured overnight at 37°C on LB liquid medium. After the culture, the cells were collected, and the plasmid was collected. A FastGene Plasmid mini kit (Nippon Genetics Co., Ltd.) was used for plasmid collection. It was confirmed by sequence analysis that there were no mutations in the nucleotide sequence of the gene inserted in the collected plasmid.

### (2) AtCPT5 gene

A full-length cDNA of AtCPT5 (At5g58780) was offered by the RIKEN BioResource Research center (Resource No. RAFL06-16-E16) via the National Bio-Resource Project.

### (Preparation of mutant gene)

### [Preparation of fragments]

To prepare a mutant gene, PCR fragments were prepared by PCR using the following primers. PCR was performed using a KOD-plus-Neo (Toyobo) in accordance with the manual. The PCR reaction involved 35 cycles with each cycle consisting of 10 seconds at 98°C, 30 seconds at 58°C, and 1 minute at 68°C.

### AtCPT5 without N-terminus (217-909)

Primer 5: Fw 5'-TCCCATCCCTACTCATGTGGCAGTG-3'
Primer 6: Rv 5'-TACAGGTTTTCCTCGAGTCAAACCCGACAGCCAA-3'

### HRT1 N-terminus (1-873) alone for fusion to AtCPT5

Primer 7: Fw 5'-ACATCACCAAGATATCATGGAATTATACAACGGTGAGAGG-3'
Primer 8: Rv 5'-CACTGCCACATGAGTAGGGATGGGA-3'

Each PCR product was electrophoresed on a 2.0% agarose gel. Then, a band in a target size was recovered from the gel using a FastGene^{™} gel-PCR extraction kit (Nippon Genetics Co., Ltd.). Overlap extension PCR was performed using the obtained PCR products as templates to prepare a chimeric sequence. The PCR reaction involved 30 cycles with each cycle consisting of 2 minutes at 94°C, 10 seconds at 98°C, 30 seconds at 55°C, and 30 seconds at 68°C.

### AtCPT5 without N-terminus + HRT1 N-terminus alone

Primer 9: Fw 5'-ACATCACCAAGATATCATGGAATTATACAACGGTGAGAGG-3'
Primer 10: Rv 5'-TACAGGTTTTCCTCGAGTCAAACCCGACAGCCAA-3'

The resulting PCR product was recovered from a 0.8% agarose gel, followed by dA addition using a 10× A-attachment mix (TOYOBO) and ligation to a pGEM-T EASY vector (Promega). *Escherichia coli* DH5α was transformed with the vector, and the transformant was applied to LB agar medium [+ 50 µg/mL Amp, + 20 µL of 50 (w/v) X-gal, + 25 µL of 100 mM IPTG] and cultured at 37°C overnight, followed by blue/white screening. Several white colonies were selected and cultured with shaking overnight at 37°C in 4 mL of LB liquid medium [+ 50 ug/mL Amp]. After the culture, the plasmid was collected using a FastGene^{™} Plasmid mini kit (Nippon Genetics Co., Ltd.) and digested with EcoRI (NEB) for 20 minutes to confirm the insertion of the insert. It was confirmed by sequence analysis that there were no mutations introduced in the PCR-amplified sequence.

### (Preparation of mutant expression vector)

A pEU-E01-MCS-TEV-His-C1 cell-free expression vector was treated with the restriction enzymes EcoRV and KpnI and then purified by gel recovery. The SLiCE solution used in a SLiCE reaction was prepared as described below.

An amount of 0.3 to 0.4 g of cultured *Escherichia coli* DH5α was gently suspended in 1.2 mL of 50 mM Tris-HCl (pH 8.0) containing 3% Triton X-100 and then incubated at room temperature for 10 minutes. The incubated suspension was centrifuged at 20000 × g for 2 minutes at 4°C, and the supernatant was collected. To the supernatant was added an equal amount of a 80% glycerol solution to prepare a SLiCE solution. The SLiCE solution was divided into aliquots and stored at -80°C until used.

### [PCR]

### Full-length AtCPT5

Primer 11: Fw 5'-ACATCACCAAGATATCATGTTGTCTATTCTCTCTTCTCTTTTATCT-3'
Primer 12: Rv 5'-TGATTGGCCGAGGCGGCCTCAAACCCGACAGCCAA-3'

### AtCPT5-N-terminal HRT1

Primer 13: Fw 5'-ACATCACCAAGATATCATGGAATTATACAACGGTGAGAGG-3'
Primer 14: Rv 5'-TACAGGTTTTCCTCGAGTCAAACCCGACAGCCAA-3'

Each obtained PCR fragment and the restriction enzyme-treated vector were mixed at a ratio of 1:1 to 3:1. To the mixture were added a 10× SLiCE buffer (500 µM Tris-HCl (pH 7.5), 100 mM MgCl₂, 10 mM ATP, 10 mM DTT) in a volume equal to one-tenth of the final volume of the reaction system and the SLiCE solution in a volume equal to one-tenth of the final volume of the reaction system, and they were reacted at 37°C for 15 minutes. Thus, pEU-AtCPT5 (Comparative Example 1) and pEU-AtCPT-N-terminal HRT1 (Example 1) were prepared.

### [Transformation of Escherichia coli]

*Escherichia coli* DH5α was transformed with each prepared vector, and the transformant was cultured on LB agar medium containing ampicillin and X-gal. Then, *Escherichia coli* cells carrying the introduced target gene were selected by colony PCR.

### [Plasmid extraction]

The *Escherichia coli* cells transformed with the plasmid containing the target gene were cultured overnight at 37°C on LB liquid medium. After the culture, the cells were collected, and the plasmid was collected. A FastGene Plasmid mini kit (Nippon Genetics Co., Ltd.) was used for plasmid collection.

### (Preparation of REF expression vector)

pGEM-REF was treated with the restriction enzymes Xho I and Bam HI and then inserted into a pEUE01-MCS-TEV-His-C1 cell-free expression vector treated similarly with the restriction enzymes Xho I and Bam HI to prepare pEU-C1-REF.

### [Transformation of Escherichia coli]

*Escherichia coli* DH5α was transformed with the prepared vector, and the transformant was cultured on LB agar medium containing ampicillin and X-gal. Then, *Escherichia coli* cells carrying the introduced target gene were selected by colony PCR.

### [Plasmid extraction]

The *Escherichia coli* cells transformed with the plasmid containing the target gene were cultured overnight at 37°C on LB liquid medium. After the culture, the cells were collected, and the plasmid was collected. A FastGene Plasmid mini kit (Nippon Genetics Co., Ltd.) was used for plasmid collection.

### (Enzyme assay)

### [Preparation of rubber particles]

Rubber particles were prepared from the latex of *Hevea brasiliensis* (*Hevea* latex) by five stages of centrifugation. To 900 mL of *Hevea* latex was added 100 mL of a 1 M Tris buffer (pH 7.5) containing 20 mM dithiothreitol (DTT) to prepare a latex solution. The latex solution was centrifuged in stages at the following different speeds: 1000 × g, 2000 × g, 8000 × g, 20000 × g, and 50000 × g. Each centrifugation stage was carried out for 45 minutes at 4°C. To the rubber particle layer left after the centrifugation at 50000 × g was added 3-[(3-cholamidopropyl)dimethylammonio]-propanesulfonate (CHAPS) to a final concentration of 0.1 to 2.0 × CMC (0.1 to 2.0 times the critical micelle concentration CMC) to wash the rubber particles. After the washing, the rubber particles were collected by ultracentrifugation (40000 × g, 4°C, 45 minutes) and re-suspended in an equal amount of a 100 M Tris buffer (pH 7.5) containing 2 mM dithiothreitol (DTT).

### [Cell-free protein synthesis reaction (Step 1: mRNA transcription reaction)]

Cell-free protein synthesis was performed using a WEPRO7240H expression kit (CellFree Sciences Co., Ltd.). An mRNA transcription reaction was performed using the vector acquired as described above as a template in accordance with the protocol of the WEPRO7240H expression kit.

### [Purification of mRNA]

After the transcription reaction, the resulting mRNA was purified by ethanol precipitation.

### [Cell-free protein synthesis reaction (Step 2: protein synthesis by dialysis)]

The following amounts of materials were added to a dialysis cup (MWCO 12000, Bio-Teck). A total amount of 60 µL of a reaction solution was prepared according to the protocol of the WEPRO7240H expression kit. To the reaction solution was added 1 to 2 mg of the rubber particles. Separately, 650 µL of SUB-AMIX was added to a No. 2 PP container (Maruemu container).

The dialysis cup was set in the No. 2 PP container, and a protein synthesis reaction was initiated at 26°C. The addition of the mRNA and the replacement of the external dialysis solution (SUB-AMIX) were performed twice after the initiation of the reaction. The reaction was carried out for 24 hours.

### [Collection of reacted rubber particles]

The solution in the dialysis cup was transferred to a new 1.5 µL tube, and the reacted rubber particles were collected by ultracentrifugation (40000 × g, 4°C, 45 minutes) and re-suspended in an equal amount of a 100 M Tris buffer (pH 7.5) containing 2 mM dithiothreitol (DTT).

### [Measurement of rubber synthesis activity of reacted rubber particles]

The rubber synthesis activity of the collected reacted rubber particles was measured as follows.

First, 50 mM Tris-HCl (pH 7.5), 2 mM DTT, 5 mM MgCl₂, 15 µM dimethylallyl diphosphate (DMAPP), 100 µM 1-14C isopentenyl diphosphate ([1-14C]IPP, specific activity 5 Ci/mol), and 10 µL of the rubber particle solution were mixed to prepare a reaction solution (100 µL in total), which was then reacted for 16 hours at 30°C.

After the reaction, 200 µL of saturated NaCl was added to the solution, and isopentenol and the like were extracted from the mixture with 1 mL of diethyl ether. Next, polyprenyl diphosphates were extracted from the aqueous phase with 1 mL of BuOH saturated with saline, and then a very long chain polyisoprenoid (rubber) was further extracted from the aqueous phase with 1 mL of toluene/hexane (1:1), followed by determination of radioactivity. The radioactivity of each phase was determined by ¹⁴C counting using a liquid scintillation counter. A higher radioactivity (dpm) indicates a higher production of the very long chain polyisoprenoid (rubber) and a higher rubber synthesis activity.

### [Measurement of molecular weight distribution of synthesized very long chain polyisoprenoid]

The molecular weight distribution of the very long chain polyisoprenoid (rubber) synthesized as above was measured under the following conditions by radio-HPLC. FIG. 1 shows the results.
HPLC system: a product of GILSON
Column: TSK guard column MP(XL) available from Tosoh Corporation, TSKgel multipore HXL-M (two columns)
Column temperature: 40°C
Solvent: THF available from Merck
Flow rate: 1 mL/min
UV detection: 215 nm
RI detection: Ramona Star (Raytest GmbH)

### (Test results)

### [Comparative Example 1 and Example 1]

FIG. 1 show the results. As shown by FIG. 1, an increase in the molecular weight of the product was demonstrated by a molecular weight distribution analysis by GPC.

The experimental results demonstrate the following: a mutant cis-prenyltransferase (CPT) family protein obtained by mutating the amino acid sequence of a N-terminal region of a cis-prenyltransferase (CPT) family protein not found on rubber particles to be identical or similar to the amino acid sequence of a N-terminal region of a cis-prenyltransferase (CPT) family protein found on rubber particles enables the production of a higher molecular weight polyisoprenoid than that produced with the unmutated cis-prenyltransferase (CPT) family protein. Then, a method for producing a polyisoprenoid using the mutant cis-prenyltransferase (CPT) family protein enables the production of a higher molecular weight polyisoprenoid than that produced with the unmutated cis-prenyltransferase (CPT) family protein.

Thus, it is also demonstrated that when a vector that contains a gene coding for the mutant cis-prenyltransferase (CPT) family protein of the first invention is introduced into a plant, the gene coding for the mutant cis-prenyltransferase (CPT) family protein of the first invention in the vector will be expressed to enable the production of a higher molecular weight polyisoprenoid in the plant as compared to before the gene recombination.

Exemplary embodiments of the present invention include:
Embodiment 1. A mutant cis-prenyltransferase (CPT) family protein, obtained by mutating an amino acid sequence of a N-terminal region of a cis-prenyltransferase (CPT) family protein not found on rubber particles to be identical or similar to an amino acid sequence of a N-terminal region of a cis-prenyltransferase (CPT) family protein found on rubber particles.
Embodiment 2. The mutant cis-prenyltransferase (CPT) family protein according to Embodiment 1,
   wherein the amino acid sequence of the N-terminal region of the cis-prenyltransferase (CPT) family protein not found on rubber particles is substituted with an amino acid sequence that is identical or similar to the amino acid sequence of the N-terminal region of the cis-prenyltransferase (CPT) family protein found on rubber particles.
Embodiment 3. The mutant cis-prenyltransferase (CPT) family protein according to Embodiment 1 or 2,
   wherein the cis-prenyltransferase (CPT) family protein found on rubber particles is a cis-prenyltransferase (CPT) family protein found on rubber particles derived from a plant of the genus *Hevea* or *Taraxacum.*
Embodiment 4. The mutant cis-prenyltransferase (CPT) family protein according to Embodiment 1 or 2,
   wherein the cis-prenyltransferase (CPT) family protein found on rubber particles is a cis-prenyltransferase (CPT) family protein found on rubber particles derived from *Hevea brasiliensis* or *Taraxacum kok-saghyz.*
Embodiment 5. The mutant cis-prenyltransferase (CPT) family protein according to any one of Embodiments 1 to 4,
   wherein the amino acid sequence of the N-terminal region of the cis-prenyltransferase (CPT) family protein not found on rubber particles is a N-terminus-containing amino acid sequence within a section from a N-terminus of the protein to a first β strand of a β sheet.
Embodiment 6. A method for producing a polyisoprenoid, the method including using the mutant cis-prenyltransferase (CPT) family protein according to any one of Embodiments 1 to 5.
Embodiment 7. The method for producing a polyisoprenoid according to Embodiment 6,
   wherein the method includes binding the mutant cis-prenyltransferase (CPT) family protein according to any one of Embodiments 1 to 5 to membrane particles *in vitro.*
Embodiment 8. A method for producing a pneumatic tire, the method including:
   producing a polyisoprenoid by the method for producing a polyisoprenoid according to Embodiment 6 or 7;
   kneading the polyisoprenoid with an additive to obtain a kneaded mixture;
   building a green tire from the kneaded mixture; and vulcanizing the green tire.
Embodiment 9. A method for producing a rubber product, the method including:
   producing a polyisoprenoid by the method for producing a polyisoprenoid according to Embodiment 6 or 7;
   kneading the polyisoprenoid with an additive to obtain a kneaded mixture;
   forming a raw rubber product from the kneaded mixture; and
   vulcanizing the raw rubber product.
Embodiment 10. A vector, containing a gene coding for the mutant cis-prenyltransferase (CPT) family protein according to any one of Embodiments 1 to 5.
Embodiment 11. The vector according to Embodiment 10, containing:
   a promoter for use in protein expression in a plant; and
   the gene coding for the mutant cis-prenyltransferase (CPT) family protein, functionally linked to the promoter.
Embodiment 12. The vector according to Embodiment 10, containing:
   a promoter having a promoter activity that drives laticifer-specific gene expression; and
   the gene coding for the mutant cis-prenyltransferase (CPT) family protein, functionally linked to the promoter.
Embodiment 13. A transgenic plant into which the vector according to any one of Embodiments 10 to 12 has been introduced.
Embodiment 14. A method for producing a polyisoprenoid in a plant by introducing the vector according to any one of Embodiments 10 to 12 into the plant.
Embodiment 15. A method for producing a pneumatic tire, the method including:
   producing a polyisoprenoid using a transgenic plant produced by introducing the vector according to any one of Embodiments 10 to 12 into a plant;
   kneading the polyisoprenoid with an additive to obtain a kneaded mixture;
   building a green tire from the kneaded mixture; and
   vulcanizing the green tire.
Embodiment 16. A method for producing a rubber product, the method including:
   producing a polyisoprenoid using a transgenic plant produced by introducing the vector according to any one of Embodiments 10 to 12 into a plant;
   kneading the polyisoprenoid with an additive to obtain a kneaded mixture;
   forming a raw rubber product from the kneaded mixture; and
   vulcanizing the raw rubber product.

### (SEQUENCE LISTING FREE TEXT)

SEQ ID NO:1: Amino acid sequence of HRT1 from *Hevea brasiliensis* 
MELYNGERPS VFRLLGKYMR KGLYSILTQG PIPTHIAFIL DGNRRFAKKH KLPEGGGHKA 60

GFLALLNVLT YCYELGVKYA TIYAFSIDNF RRKPHEVQYV MDLMLEKIEG MIMEESIINA 120

YDICVRFVGN LKLLSEPVKT AADKIMRATA NNSKCVLLIA VCYTSTDEIV HAVEESSELN 180

SNEVCNNQEL EEANATGSGT VIQIENMESY SGIKLVDLEK NTYINPYPDV LIRTSGETRL 240

SNYLLWQTTN CILYSPHALW PEIGLRHVVW AVINFQRHYS YLEKHKEYLK 290
SEQ ID NO:2: Nucleotide sequence of gene coding for HRT1 from *Hevea brasiliensis* 
atggaattat acaacggtga gaggccaagt gtgttcagac ttttagggaa gtatatgaga 60

aaagggttat atagcatcct aacccagggt cccatcccta ctcatattgc cttcatattg 120

gatggaaaca ggaggtttgc taagaagcat aaactgccag aaggaggtgg tcataaggct 180

ggatttttag ctcttctgaa cgtactaact tattgctatg agttaggagt gaaatatgcg 240

actatctatg cctttagcat cgataatttt cgaaggaaac ctcatgaggt tcagtacgta 300

atggatctaa tgctggagaa gattgaaggg atgatcatgg aagaaagtat catcaatgca 360

tatgatattt gcgtacgttt tgtgggtaac ctgaagcttt taagtgagcc agtcaagacc 420

gcagcagata agattatgag ggctactgcc aacaattcca aatgtgtgct tctcattgct 480

gtatgctata cttcaactga tgagatcgtg catgctgttg aagaatcctc tgaattgaac 540

tccaatgaag tttgtaacaa tcaagaattg gaggaggcaa atgcaactgg aagcggtact 600

gtgattcaaa ttgagaacat ggagtcgtat tctggaataa aacttgtaga ccttgagaaa 660

aacacctaca taaatcctta tcctgatgtt ctgattcgaa cttctgggga gacccgtctg 720

agcaactact tactttggca gactactaat tgcatactgt attctcctca tgcactgtgg 780

ccagagattg gtcttcgaca cgtggtgtgg gcagtaatta acttccaacg tcattattct 840

tacttggaga aacataagga atacttaaaa taa 873
SEQ ID NO:3: Amino acid sequence of AtCPT5 from *Arabidopsis thaliana* 
MLSILSSLLS LLFLFIISCF FITSHFWFPL SLPKILGFIK ITSSRDDYDN EQRDEGTYVV 60

GVEELQRELM PRHVAVIMDG NRRWAKRAGL LTSQGHEAGA KRLIEFSELC FKLGIHTVSA 120

FAFSTENWGR HKIEVKCLMS LIQHYLKSKI QYFQREETRV SVIGNLTKIP ESLLRTVQEI 180

EEATRSYKKK HLILAIDYSG RLDILRACKS IVKKSEKGLI REEDVDEALI ERELLTNCTE 240

FPSPDLLIRT SGEQRISNFF LWQLAYTELF FSPVLWPDFD KDKLLEALVS YQRRERRFGC 300

RV 302
SEQ ID NO:4: Amino acid sequence of HbCPT5 from *Hevea brasiliensis* 
MEIFEAGRPS VLASLGRFIR KCIFRILSIG PIPSHVAFIM DGNRRFAKKE KLEEGSGHRA 60

GSLALMSTLK YCYELGVKYV TIYAFSIDNF RRRPDEVQLI MDLILEKIEG LLRDENVVNA 120
 
YGIRVRFVGT LKLLSEPVRV AAEKVARASA KNTKFVLVIC IAYSSTDEIV HAVQESCKYK 180

LNKIEPSNSN RACNDANEQV EENGKKIDST ITHGVQESCK DETDKSRTIN AKPMYNGVTK 240

EAGGTDNANT VIVNSIGDKW DDAHELEATR TGNGVISVEE IDKMLSHSSI KLVDIEKKLH 300

MAVAPDPDIL VRTSGESRLS NFLLWQTSNC SLYSPKALWP EIGLRHLVWA VITFQRNHSY 360

LEKKKQL 368
SEQ ID NO:5: Nucleotide sequence of gene coding for AtCPT5 from *Arabidopsis thaliana* 
atgttgtcta ttctctcttc tcttttatct ctcctttttc tgtttatcat ctcttgtttc 60

ttcatcacaa gccatttttg gttccctctt tccttgccaa aaatactcgg attcatcaaa 120

atcacatctt cgagagacga ttatgacaac gagcaacgtg acgagggaac ttatgtggta 180

ggagtggagg agctacaaag agagctgatg ccaagacatg tggcagtgat aatggacgga 240

aaccggagat gggccaaacg ggccggattg ctgacgtcac aaggccacga ggccggagct 300

aaacggctta tagagttctc cgagctttgc tttaaattgg ggattcatac agtttcagct 360

tttgccttct ccacagagaa ttggggaaga cacaagattg aggttaagtg cttgatgtct 420

ttgatccaac attacctcaa gtccaagatc caatatttcc aaagagagga aactcgagtt 480

tctgttatcg ggaacctaac gaagatccct gagtctctcc tccgaacagt ccaagagata 540

gaggaagcta cgagaagcta taagaagaag catctcatat tggcaataga ttacagcggg 600

agattagaca tcttgcgagc ttgcaagagt attgtgaaga aatcagaaaa agggttgatc 660

cgagaggaag atgtagacga ggcattgatc gaaagagagc ttctgacaaa ttgtactgag 720

ttcccaagtc ctgatctatt gattaggaca agtggagaac agaggattag taacttcttc 780

ttgtggcaac ttgcttatac agagctcttc ttctcgccgg tcctttggcc tgatttcgat 840

aaggataagc ttctagaggc cctggtttcg tatcagcgcc gggaaagacg atttggctgt 900

cgggtttga 909
SEQ ID NO:6: Nucleotide sequence of gene coding for HbCPT5 from *Hevea brasiliensis* 
atggaaatat ttgaggctgg taggccaagc gtgcttgcaa gtttggggag atttatcaga 60

aaatgcatat ttcgcattct atcaataggt cccatcccaa gtcatgttgc cttcataatg 120

gatggaaacc ggaggtttgc taagaaggag aaactggaag aaggatctgg tcacagggct 180

ggatctttag ctctaatgtc cacgcttaag tactgctatg agttgggagt gaagtatgtg 240

actatatatg ccttcagcat tgataatttt agaaggcgac ctgatgaggt tcagctcata 300

atggatctaa tactggagaa gattgagggg ctgctcaggg acgaaaatgt tgtcaatgca 360

tatggcatca gagtacgatt tgtaggtacc ttgaagcttc taagtgagcc agtcagggtc 420

gcagcagaaa aagtcgccag ggctagtgcc aagaatacca agtttgtgct tgtcatttgt 480
 
atagcctatt cttcaactga tgagattgtg catgctgttc aagaatcttg taaatataaa 540
ttgaacaaaa ttgagccatc taactccaac agggcttgca atgatgcgaa tgaacaagta 600
gaagaaaatg gtaagaagat agatagtacc atcacacatg gtgttcaaga atcctgcaaa 660
gatgaaacag ataaatctcg cacaataaac gcaaagccaa tgtataatgg tgtgaccaaa 720
gaagctggag ggactgacaa tgctaatact gtgatagtaa attccatcgg agacaagtgg 780
gatgatgctc acgaactgga ggcaactaga actggcaatg gtgtgatttc agtagaagaa 840
attgataaga tgctgtcaca ttctagcata aagctggtag acattgagaa aaaattgcac 900
atggctgtag cccctgatcc tgatatcttg gttcgaactt ctggagagag ccgtctgagc 960
aacttcctac tttggcagac tagtaactgt tcactgtatt ctccaaaggc actgtggccg 1020
gagattggcc tacgccactt ggtgtgggca gtaataacct tccaacgtaa tcattcttat 1080
ttggagaaga aaaagaagca gttgtaa 1107
SEQ ID NO:7: Amino acid sequence of AtCPT5-N-terminal HRT1 
MELYNGERPS VFRLLGKYMR KGLYSILTQG PIPTHVAVIM DGNRRWAKRA GLLTSQGHEA 60

GAKRLIEFSE LCFKLGIHTV SAFAFSTENW GRHKIEVKCL MSLIQHYLKS KIQYFQREET 120

RVSVIGNLTK IPESLLRTVQ EIEEATRSYK KKHLILAIDY SGRLDILRAC KSIVKKSEKG 180

LIREEDVDEA LIERELLTNC TEFPSPDLLI RTSGEQRISN FFLWQLAYTE LFFSPVLWPD 240

FDKDKLLEAL VSYQRRERRF GCRV 264
SEQ ID NO:8: Nucleotide sequence of gene coding for AtCPT5-N-terminal HRT1 
atggaattat acaacggtga gaggccaagt gtgttcagac ttttagggaa gtatatgaga 60

aaagggttat atagcatcct aacccagggt cccatcccta ctcatgtggc agtgataatg 120

gacggaaacc ggagatgggc caaacgggcc ggattgctga cgtcacaagg ccacgaggcc 180

ggagctaaac ggcttataga gttctccgag ctttgcttta aattggggat tcatacagtt 240

tcagcttttg ccttctccac agagaattgg ggaagacaca agattgaggt taagtgcttg 300

atgtctttga tccaacatta cctcaagtcc aagatccaat atttccaaag agaggaaact 360

cgagtttctg ttatcgggaa cctaacgaag atccctgagt ctctcctccg aacagtccaa 420

gagatagagg aagctacgag aagctataag aagaagcatc tcatattggc aatagattac 480

agcgggagat tagacatctt gcgagcttgc aagagtattg tgaagaaatc agaaaaaggg 540

ttgatccgag aggaagatgt agacgaggca ttgatcgaaa gagagcttct gacaaattgt 600

actgagttcc caagtcctga tctattgatt aggacaagtg gagaacagag gattagtaac 660

ttcttcttgt ggcaacttgc ttatacagag ctcttcttct cgccggtcct ttggcctgat 720

ttcgataagg ataagcttct agaggccctg gtttcgtatc agcgccggga aagacgattt 780

ggctgtcggg tttga 795
SEQ ID NO:9: Nucleotide sequence of gene coding for REF from *Hevea brasiliensis* 
atggctgaag acgaagacaa ccaacaaggg cagggggagg ggttaaaata tttgggtttt 60

gtgcaagacg cggcaactta tgctgtgact accttctcaa acgtctatct ttttgccaaa 120

gacaaatctg gtccactgca gcctggtgtc gatatcattg agggtccggt gaagaacgtg 180

gctgtacctc tctataatag gttcagttat attcccaatg gagctctcaa gtttgtagac 240

agcacggttg ttgcatctgt cactattata gatcgctctc ttcccccaat tgtcaaggac 300

gcatctatcc aagttgtttc agcaattcga gctgccccag aagctgctcg ttctctggct 360

tcttctttgc ctgggcagac caagatactt gctaaggtgt tttatggaga gaattga 417
SEQ ID NO:10: Amino acid sequence of REF from *Hevea brasiliensis* 
MAEDEDNQQG QGEGLKYLGF VQDAATYAVT TFSNVYLFAK DKSGPLQPGV DIIEGPVKNV 60

AVPLYNRFSY IPNGALKFVD STVVASVTII DRSLPPIVKD ASIQVVSAIR AAPEAARSLA 120

SSLPGQTKIL AKVFYGEN 138
SEQ ID NO:11: Primer 1
   tttggatccg atggaattat acaacggtga gagg 34
SEQ ID NO:12: Primer 2
   tttgcggccg cttattttaa gtattcctta tgtttctcc 39
SEQ ID NO:13: Primer 3
   tttctcgaga tggctgaaga cgaagac 27
SEQ ID NO:14: Primer 4
   tttggatcct caattctctc cataaaac 28
SEQ ID NO:15: Primer 5
   tcccatccct actcatgtgg cagtg 25
SEQ ID NO:16: Primer 6
   tacaggtttt cctcgagtca aacccgacag ccaa 34
SEQ ID NO:17: Primer 7
   acatcaccaa gatatcatgg aattatacaa cggtgagagg 40
SEQ ID NO:18: Primer 8
   cactgccaca tgagtaggga tggga 25
SEQ ID NO:19: Primer 9
   acatcaccaa gatatcatgg aattatacaa cggtgagagg 40
SEQ ID NO:20: Primer 10
   tacaggtttt cctcgagtca aacccgacag ccaa 34
SEQ ID NO:21: Primer 11
   acatcaccaa gatatcatgt tgtctattct ctcttctctt ttatct 46
SEQ ID NO:22: Primer 12
   tgattggccg aggcggcctc aaacccgaca gccaa 35
SEQ ID NO:23: Primer 13
   acatcaccaa gatatcatgg aattatacaa cggtgagagg 40
SEQ ID NO:24: Primer 14
   tacaggtttt cctcgagtca aacccgacag ccaa 34

## Claims

1. A mutant cis-prenyltransferase (CPT) family protein, obtained by mutating an amino acid sequence of a N-terminal region of a cis-prenyltransferase (CPT) family protein not found on rubber particles to be identical or similar to an amino acid sequence of a N-terminal region of a cis-prenyltransferase (CPT) family protein found on rubber particles.

2. The mutant cis-prenyltransferase (CPT) family protein according to claim 1,
wherein the amino acid sequence of the N-terminal region of the cis-prenyltransferase (CPT) family protein not found on rubber particles is substituted with an amino acid sequence that is identical or similar to the amino acid sequence of the N-terminal region of the cis-prenyltransferase (CPT) family protein found on rubber particles.

3. The mutant cis-prenyltransferase (CPT) family protein according to claim 1 or 2,
wherein the cis-prenyltransferase (CPT) family protein found on rubber particles is a cis-prenyltransferase (CPT) family protein found on rubber particles derived from a plant of the genus *Hevea* or *Taraxacum.*

4. The mutant cis-prenyltransferase (CPT) family protein according to claim 1 or 2,
wherein the cis-prenyltransferase (CPT) family protein found on rubber particles is a cis-prenyltransferase (CPT) family protein found on rubber particles derived from *Hevea brasiliensis* or *Taraxacum kok-saghyz.*

5. The mutant cis-prenyltransferase (CPT) family protein according to any one of claims 1 to 4,
wherein the amino acid sequence of the N-terminal region of the cis-prenyltransferase (CPT) family protein not found on rubber particles is a N-terminus-containing amino acid sequence within a section from a N-terminus of the protein to a first β strand of a β sheet.

6. A method for producing a polyisoprenoid, the method comprising using the mutant cis-prenyltransferase (CPT) family protein according to any one of claims 1 to 5.

7. The method for producing a polyisoprenoid according to claim 6,
wherein the method comprises binding the mutant cis-prenyltransferase (CPT) family protein according to any one of claims 1 to 5 to membrane particles *in vitro.*

8. A method for producing a pneumatic tire, the method comprising:
producing a polyisoprenoid by the method for producing a polyisoprenoid according to claim 6 or 7;
kneading the polyisoprenoid with an additive to obtain a kneaded mixture;
building a green tire from the kneaded mixture; and
vulcanizing the green tire.

9. A method for producing a rubber product, the method comprising:
producing a polyisoprenoid by the method for producing a polyisoprenoid according to claim 6 or 7;
kneading the polyisoprenoid with an additive to obtain a kneaded mixture;
forming a raw rubber product from the kneaded mixture; and
vulcanizing the raw rubber product.

10. A vector, comprising a gene coding for the mutant cis-prenyltransferase (CPT) family protein according to any one of claims 1 to 5.

11. The vector according to claim 10, comprising:
a promoter for use in protein expression in a plant; and
the gene coding for the mutant cis-prenyltransferase (CPT) family protein, functionally linked to the promoter.

12. The vector according to claim 10, comprising:
a promoter having a promoter activity that drives laticifer-specific gene expression; and
the gene coding for the mutant cis-prenyltransferase (CPT) family protein, functionally linked to the promoter.

13. A transgenic plant into which the vector according to any one of claims 10 to 12 has been introduced.

14. A method for producing a polyisoprenoid in a plant by introducing the vector according to any one of claims 10 to 12 into the plant.

15. A method for producing a pneumatic tire, the method comprising:
producing a polyisoprenoid using a transgenic plant produced by introducing the vector according to any one of claims 10 to 12 into a plant;
kneading the polyisoprenoid with an additive to obtain a kneaded mixture;
building a green tire from the kneaded mixture; and
vulcanizing the green tire.

16. A method for producing a rubber product, the method comprising:
producing a polyisoprenoid using a transgenic plant produced by introducing the vector according to any one of claims 10 to 12 into a plant;
kneading the polyisoprenoid with an additive to obtain a kneaded mixture;
forming a raw rubber product from the kneaded mixture; and
vulcanizing the raw rubber product.
